(19) Europäisches Patentamt
European Patent Office
Office européen des brevets

(11) Publication number: **0 472 573 B1**

(12) **EUROPEAN PATENT SPECIFICATION**

(45) Date of publication of patent specification: **27.12.95**

(51) Int. Cl.[6]: **A01H 1/06**, C12N 15/60

(21) Application number: **90907271.2**

(22) Date of filing: **16.05.90**

(86) International application number:
**PCT/GB90/00753**

(87) International publication number:
**WO 90/14000 (29.11.90 90/27)**

(54) **HERBICIDE RESISTANT MAIZE**

(30) Priority: **17.05.89 GB 8911295**
**21.07.89 GB 8916725**
**21.07.89 GB 8916726**
**21.07.89 GB 8916727**
**21.07.89 GB 8916728**
**21.07.89 GB 8916729**
**21.07.89 GB 8916730**

(43) Date of publication of application:
**04.03.92 Bulletin 92/10**

(45) Publication of the grant of the patent:
**27.12.95 Bulletin 95/52**

(84) Designated Contracting States:
**AT BE CH DE DK ES FR GB IT LI LU NL SE**

(56) References cited:
**EP-A- 0 154 204**

**Maydica XXIII, 1978, M.G. NEUFFER et al.: "Paraffin Oil Technique for TreatingMature Corn Pollen with Chemical Mutagens", pages 21-28**

(73) Proprietor: **ZENECA LIMITED**
**15 Stanhope Gate**
**London W1Y 6LN (GB)**

(72) Inventor: **BRIGHT, Simon William Jonathan**
**24 Pound lane**
**Marlow,**
**Bucks SL7 2AY (GB)**
Inventor: **CHANG, Ming Tang**
**1419 Illinois Avenue**
**Ames, IA 50010 (US)**
Inventor: **EVANS, Ian Jeffrey**
**38 Wilmington Close,**
**Woodley**
**Reading,**
**Berkshire RG5 4LR (GB)**
Inventor: **MACDONALD, Mairi Jean**
**35 Flamingo Close**
**Wokingham,**
**Berkshire RG11 25J (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, Volume 112, No. 3, 15 January 1990, (Columbus, Ohio, US),E.B. SWANSON et al.: "Microspore Mutagenesis and Selection: Canola Plants withField Tolerance to the Imidazolinones", see page 304 * Abstract 18959k, &Theor. Appl. Genet. 1989, 78(4), 525-30***

(74) Representative: **Huskisson, Frank Mackie et al**
**Intellectual Property Group,**
**Zeneca Seeds,**
**Jealott's Hill Research Station,**
**PO Box No 3539**
**Bracknell,**
**Berkshire RG12 6YD (GB)**

## Description

This invention relates to herbicide resistant maize plants and the seed and progeny thereof.

The purpose in providing crop plants which resist the action of a herbicide is to facilitate the destruction of weeds growing between the plants by the overall application of a herbicidally effective concentration of a herbicide which would destroy the crop plant in its normal, that is herbicide sensitive, state. Such resistant plants are also useful for use in a locus of any short term carry-over of herbicide from a previous crop.

Methods are known by which populations of plants may be obtained which contain a great number of random mutations. Such methods include tissue culture techniques where spontaneous somaclonal variation occurs in the presence or absence of a mutagen. By applying to the cultures some form of selection pressure it is possible to recover cells which resist that pressure. Depending on the plant species it is sometimes possible to regenerate whole plants from the resistant cells. Such tissue culture selection methods have been used in the past to select for resistance to herbicides.

Neuffer and Coe [Maydica XXIII (1978) 21-28; page 21] have described a procedure for corn (maize) pollen mutagenesis using mutagens suspended in light paraffin oil, followed by pollination of a recipient plant with the mutagenized pollen. As reported, there is no indication that any attempt was ever made to locate and isolate commercially important mutants and, although it is said that the resulting plants were examined for mutants, no details are given as to the properties of any mutants which may have been found, probably by visual inspection.

In Plant Breeding Reviews 5, pages 39 to 180, Bird and Neuffer review the uses of mutagenic processes to produce variation in maize. Pollen mutagenesis is discussed at pages 150 and 151. Pollen mutageneseis gives a relatively high frequency of variation in the $M_1$ generation compared with other available procedures. However, no suggestion is made in respect of the use of pollen mutagenesis for generation of mutants which display resistance to herbicide action. Section III (page 149) describes some of the difficulties of the use of mutagenesis as a source of genetic variation. One important aspect is the method by which the population of putative mutants is screened for useful phenotypes.

An object of the present invention is to provide herbicide resistant maize.

According to the present invention there is provided a method of producing mutant Zea mays possessing resistance to a normally lethal concentration of a chosen herbicide, comprising subjecting isolated pollen of Zea mays to the action of a chemical mutagen, recovering mutagenised pollen, pollinating a female parent therewith, harvesting seed from the mature plant, growing the seeds under selection pressure of the chosen herbicide or a second herbicide having the same mode of action as the chosen herbicide, and recovering progeny resistant to the applied selection pressure.

Preferably the chemical mutagen is ethyl methanesulphonate.

Preferably the herbicide utilised for applying selection pressure is an inhibitor of acetolactate synthase and more preferably is imazethapyr.

Preferably also the selection pressure is applied by application of the herbicide to the seeds pre-emergence.

It is preferred that the concentration of herbicide used for selection pressure is insufficient to inhibit germination but sufficient to inhibit growth of sensitive plants.

Seeds obtained by the method to the present invention have been deposited at the National Collection of Industrial & Marine Bacteria, Aberdeen, United Kingdom, official details of the deposits being as in Table 1 below.

TABLE 1

| Mutant Number | Applicant Code | Accession Number | Date of Deposit |
|---|---|---|---|
| 1 | ICI/88/01 63-3D | NCIMB 40137 | 8 May 1989 |
| 2 | ICI/88/03 56-3G | NCIMB 40136 | 8 May 1989 |
| 3 | ICI/88/04 82-5H | NCIMB 40138 | 8 May 1989 |
| 4 | see footnote | | |
| 5 | ICI/88/07 97-4L | NCIMB 40167 | 18 July 1989 |
| 6 | ICI/88/07 76-6H | NCIMB 40168 | 18 July 1989 |
| 7 | ICI/88/07 58-5D | NCIMB 40169 | 18 July 1989 |
| 8 | ICI/88/09 77-7N | NCIMB 40170 | 18 July 1989 |
| 9 | ICI/88/10 1-4C | NCIMB 40171 | 18 July 1989 |
| 10 | ICI/88/10 72-4N | NCIMB 40172 | 18 July 1989 |

* Mutant Number 4 was withdrawn in the light of further testing which showed that it possessed no useful level resistance. The number has been retained in order to retain the sequence of numbers in the description which follows.

Each of these deposits is a genetic mixture, segregating mutant and non-mutant seeds. The mutants are heterozygous for the gene conferring herbicide resistance. Mutant plants may be derived from these seeds by growing under the conditions described below under the heading "Screening" in the presence of the herbicide imazethapyr.

The seeds prepared by the invention may be used for the production of herbicide resistant hybrids by crossing of plants grown from the seeds with other maize plant lines.

The plants of this invention are known to be resistant to certain members of the imidazolinone family of herbicides, for example, imazethapyr [5-ethyl-2-(5-iso- propyl-5-methyl-4-oxo-2-imidazolin-2-yl) nicotinic acid: (Trade Mark PURSUIT, American Cyanamid)]. Cross resistance to herbicides of the sulphonylurea family, chlorsulfuron, for example, has also been found, as has resistance to the phenoxy-pyrimidines and the triazolopyrimidines. Resistance to other, as yet untested, herbicide families may also be present.

The herbicides against which the plants produced by the invention have so far been tested are those whose mode of action is inhibition of the enzyme acetolactate synthase (ALS), also known as acetohydroxyacid synthase (AHAS). It is convenient to refer to the herbicides by the Trade Marks by which they are sold commercially. Figure 2 of the drawings gives the chemical structures of the active ingredients.

A detailed exposition of the molecular basis of the resistance to the sulphonylurea herbicides is given in European Patent Application 257,993 (E.I. Du Pont de Nemours and Company). Isolation of imidazolinone-resistant maize from tissue culture is reported in United States Patent Number 4,761,373 (Molecular Genetics Inc.). Various plant species and several herbicides are reported in the literature as having been used in tissue culture processes to isolate resistant mutants.

In comparison, the present invention does not utilise tissue culture. The resistance is introduced by mutagenesis of pollen by a chemical mutagen. Selection is effected directly on the seeds formed after fertilisation with the mutagenised pollen either by treatment of the seed pre-emergence or at the seedling stage, or both.

Certain advantages accrue from the use of pollen mutagenesis as a method of creating mutants rather than the more usual method of relying on somaclonal variation to produce the variation. In order that somaclonal variation may occur it is required that a culture of plant tissue be established. This requirement restricts the choice of genotype which may be used as it is not always possible to regenerate whole plants from the cultured tissue. On the other hand, mutagenised pollen may be applied to any recipient maize genotype, including commercially important and well-established elite breeding lines. Also the rate of occurrence of undesirable mutations which somaclonal variation is known to produce is reduced.

In this invention, selection is carried out at at the $M_1$ generation, with the result that only dominant mutations are selected. Also, being carried out on whole plants or on the seed pre-emergence, or both, allows the herbicide concentration to mimic the field conditions more closely than is possible with the application of the selection pressure of the herbicide to a tissue culture. In the tissue culture selection method, whole plants have to be regenerated from the tissue and grown to maturity before any indication of the performance of the progeny under field application rates of the herbicide can be obtained. In our method, selection is made directly on the plants under concentrations of herbicide which are comparable to those which are recommended for normal weed-killing activity in the field. Selection on the $M_2$ generation,

as with the tissue culture method, selects recessive mutations as well as dominant traits. Dominance of a desirable trait is generally viewed as more useful and easier to handle in a breeding programme especially of hybrid crops.

We believe that the mutants selected under herbicide pressure vary according to the particular member of the herbicide family which is used. All of our mutants were selected under pressure of imazethapyr. Had a different imidazolinone herbicide been used, a different spectrum of mutants would have been selected.

We have found, quite surprisingly, that the mutants which we have isolated by the method of the invention are free of deleterious mutations. This was entirely unexpected and the reason for this advantage is not entirely clear. We believe, but do not wish to be bound by this explanation, that the degree of control which we are able to exercise over the selection step, using carefully controlled rates of application of the herbicide , for example, giving a good overall and uniform rate of exposure, may be responsible.

The invention will now be described by the following summary of the method by which the herbicide-resistant plants of the invention were derived.

The Figures which accompany this application are as follows:

Figure 1 is a flow-chart showing the derivation of several generations of progeny from plants generated by this invention;

Figure 2 shows the chemical structures of the herbicides used in this invention;

Figure 3 is a graph of ALS enzyme activity in the presence of imazethapyr. The enzyme is extracted from plants heterozygous for the resistance gene;

Figure 4 is a graph of ALS enzyme activity in the presence of imazapyr. The enzyme is extracted from plants heterozygous for the resistance gene;

Figure 5 is a graph of ALS enzyme activity in the presence of imazaquin. The enzyme is extracted from plants heterozygous for the resistance gene;

Figure 6 is a graph of ALS enzyme activity in the presence of chlorsulfuron. The enzyme is extracted from plants heterozygous for the resistance gene;

Figure 7 is a graph of ALS enzyme activity in the presence of chlorimuron. The enzyme is extracted from plants heterozygous for the resistance gene;

Figure 8 is a graph of ALS enzyme activity in the presence of thiacarburon. The enzyme is extracted from plants heterozygous for the resistance gene;

Figure 9 is an enzyme activity graph of the activity of ALS extracted from leaves of progeny of mutants 1 and 2 which are homozygous for the resistant allele and in the presence of imazethapyr (PURSUIT);

Figure 10 is an enzyme activity graph of the activity of ALS extracted from leaves of mutants 1 and 2 which are homozygous for the resistant allele and in the presence of imazaquin (SCEPTER);

Figure 11 is an enzyme activity graph of the activity of ALS extracted from leaves of mutants 1 and 2 which are homozygous for the resistant allele and in the presence of imazapyr (ARSENAL);

Figure 12 is an enzyme activity graph of the activity of ALS extracted from leaves of mutants 1 and 2 which are homozygous for the resistant allele and in the presence of chlorsulfuron (GLEAN);

Figure 13 is an enzyme activity graph of the activity of ALS extracted from leaves of mutants 1 and 2 which are homozygous for the resistant allele and in the presence of chlorimuron (CLASSIC);

Figure 14 is an enzyme activity graph of the activity of ALS extracted from leaves of mutants 1 and 2 which are homozygous for the resistant allele and in the presence of thiacarburon (HARMONY);

Figure 15 is an enzyme activity graph of the activity of ALS extracted from leaves of mutants 1 and 2 which are homozygous for the resistant allele and in the presence of a triazolopyrimidine;

Figure 16 is an enzyme activity graph of the activity of ALS extracted from leaves of mutants 1 and 2 which are homozygous for the resistant allele and in the presence of a phenoxypyrimidine;

Figure 17 is a dose response curve for the herbicide imazethapyr (PURSUIT);

Figure 18 is a dose response curve for the herbicide imazaquin (SCEPTER);

Figure 19 is a dose response curve for the herbicide chlorimuron (CLASSIC);

Figure 20 is a dose response curve for the herbicide chlorsulfuron (GLEAN);

Figure 21 is a dose response curve for a triazolopyrimidine herbicide;

Figure 22 is a dose response curve for a phenoxypyrimidine herbicide; and,

Figure 23 is a dose response curve for the herbicide imazethapyr (PURSUIT) for mutants 1 and 2 in both heterozygous and homozygous forms.

## 1. PRODUCTION OF M1 SEED

A stock solution of ethyl methane sulphonate (EMS) was made up to contain one millilitre of EMS in 100 ml of light paraffin oil. The stock solution was stored under refrigeration.

Fresh pollen grains with anthers were harvested from a total of twenty tassels of field grown maize inbred line UE95. Pollen grains were separated from the anthers using a Glassine (Trade Mark) bag.

Around 3 milligrams of pollen were added to 45 millilitres of the EMS stock solution in a 60 ml capacity bottle. The pollen/EMS solution mixture was shaken vigorously for 30 seconds then shaken four or five times every three minutes over a period of 40 minutes, to prevent precipitation of the pollen grains. The treated pollen grains were brushed on to the silks of the detasseled inbred female parent (coded UE95).

The plants were grown to maturity and the M1 seeds harvested.

2. SCREENING

M1 seed was sown, 100 seeds per tray, in WCB growing medium (low organic matter, 45% loam, 55% grit) and sprayed to 'run-off' using a track sprayer, with a solution of imazethapyr (PURSUIT) at a concentration calculated to be the equivalent of 250 g/ha of active ingredient . The seeds were covered with 0.5 inch of WCB and grown in the glasshouse at 25 ° C.

The chosen application rate was such that germination was close to 100%, but subsequently all susceptible plants were severely affected. However, after about two weeks, the initial effect of the herbicide became apparent: thin striped leaves and reduced height of only about 20% of the height of the control plants. After three to four weeks almost all the sensitive plants were completely dead. Unsprayed UE95 plants were always grown in parallel with each screen as a control as it was already known that normal and M1 seedlings of UE95 are almost indistinguishable when germinated and grown to maturity without spraying.

3. SELECTION AND GROWTH

A total of ten plants (representing 0.01% of the total) were initially selected from the screen as exhibiting tolerance of normally lethal dosages of the herbicide. Mutant No.4 was subsequently found to be of the sensitive phenotype and was withdrawn from the programme. Of the remaining nine, the majority were morphologically similar to the untreated control plants after selection but four were affected, being shorter and exhibiting other herbicidal effects. Samples of seeds of these nine plants back-crossed to the parent UE95 (Mutants 1 to 10, excluding No.4) are those which have been deposited with the National Collection of Industrial and Marine Bacteria (see Table 1 above).

4. RFLP STUDY

In order to confirm that the sibling plants were indeed of UE95 genotype in origin and not an intrinsically more resistant contaminating inbred or hybrid, RFLP (restriction fragment length polymorphism) fingerprinting analysis was performed on DNA extracted from leaf tissue from all ten plants.

Approximately 1 to 5 grams of leaf tissue was removed from each plant (ranging in age from four weeks to 'mature'), DNA extracted and RFLP analysis performed using diagnostic single copy probes.

RFLP analysis confirmed that the selected plants were of the UE95 genotype.

5. SEGREGATION STUDY

Resistant plants were used in reciprocal back-crosses with homozygous, herbicide sensitive UE95. The frequency of resistant progeny in the $M_1BC_1$ or $M_1BC_2$ generations was found by treatment with imazethapyr and counting the survivors. The results are shown in Table 2 below, along with the calculated value of $\chi^2$ (for a 1:1 ratio).

As can been seen from the figures quoted in Table 2, the ratio of resistant to sensitive plants in the progeny of the backcross is not significantly different from 1:1 for all of the mutants except numbers 3 and 7, indicating that resistance is controlled by a single dominant gene.

TABLE 2

| Mutant No. | Number tested | Number sensitive | Number resistant | $\chi^2$ (for 1:1) |
|---|---|---|---|---|
| 1 | 40 | 18 | 22 | 0.40 |
| 2 | 228 | 110 | 118 | 0.28 |
| 3* | 206 | 120 | 86 | 5.61 |
| 5 | 168 | 94 | 74 | 2.38 |
| 6 | 60 | 36 | 24 | 2.40 |
| 7* | 168 | 139 | 29 | 72.00 |
| 8 | 188 | 88 | 100 | 0.77 |
| 9 | 168 | 83 | 85 | 0.02 |
| 10 | 134 | 73 | 71 | 0.03 |

* The resistance of these mutants to imazethapyr is low and it was therefore difficult to make a meaningful assessment in this experiment but, experience in other tests indicates that segregation is in the region of 1:1.

6. GENERATION OF F1 SEED FROM RESISTANT PLANTS

All ten plants identified in the screen were used in reciprocal sib-crossing with UE95 plants to give seed (designated $M_1BC_1$), that is, the resistant plants were used as both male and female donors.

7. RESCREENING OF THE PROGENY SEED

From each resulting cob, a small sample of seed was screened for imazethapyr (PURSUIT) resistance, employing the same conditions as are described above for the initial screen.

Resistant and sensitive phenotypes segregated in the progeny of each resistant plant.

8. PRODUCTION OF FURTHER GENERATIONS

Referring to Figure 1, the $M_1BC_1$ plants were self pollinated to give generation $M_1BC_1S_1$ which was again self-pollinated to give $M_1BC_1S_2$. From that generation it was possible to identify, by the fact that the resistant trait is non-segregating, lines which are homozygous for the trait. These homozygous lines derived from Mutant No.1 may be utilised for the production of F1 hybrids which possess resistance to the imidazolinone herbicides or for further breeding work.

9. ENZYME ASSAYS

Seeds of generation $M_1BC_1$ $M_1BC_2$ of the nine mutants were sprayed pre-emergence with the herbicide imazethapyr (PURSUIT) (Trade Mark) at a rate of 250 g/ha and grown in a growth chamber (16 hour day, 27°C; 8 hour night, 17°C). Plants were harvested after 11 days.

Four grams of leaf material were harvested from just above the first leaf axis and ground in a mortar and pestle in 20ml of a solution containing 40mM Tricine (Trade Mark), 10mM EDTA, 5mM pyruvate, 80 µM flavin adenine dinucleotide (FAD), 1mM dithiothreitol (DTT), pH 8, plus 0.8g Polyclar AT. The homogenate was pressed through four layers of muslin and centrifuged at 30,000g for 20 minutes.

The supernatant was brought to 65% saturation with ammonium sulphate, left to precipitate for 30 minutes and then centrifuged at 30.000g for 20 minutes. The pellet was resuspended in 2.5ml of 40mM Tricine, 10mM EDTA, 5mM pyruvate, 80µM FAD, 25% (v/v) glycerol, 1mM DTT, pH 8, and desalted into 3.5ml of 40mM Tricine, 10mM EDTA, 25% (v/v) glycerol, 1mM DTT, pH 8.

One hundred microlitres of the enzyme extract was used for each assay. Final reagent concentrations were: 120mM Tricine, 50mM pyruvate, 10mM $MgCl_2$, 66mM FAD, 93µM thiamine pyrophosphate (TPP), pH 8, in the presence or absence of a herbicide of interest, in a volume of 750 µl. Incubations were carried out at 37°C for 30 minutes. Reactions were stopped with 250µl of 1.84M sulphuric acid, and decarboxylation of the acetolactate carried out at 37°C for 75 minutes, or at 60°C for 30 minutes. Assay blanks had sulphuric acid added prior to the enzyme extract. To the samples and blanks 650µl of α-naphthol in 2.73M NaOH/0.16%(w/v) creatine were added followed by incubation at 37°C for 30 minutes. After centrifugation at 30,000g the optical density of the supernatant was read at 540nm.

The assay procedure described was carried out on ALS enzyme extracted from each of the nine mutants for the following herbicides:

imazethapyr (PURSUIT)
imazapyr (ARSENAL)
imazaquin (SCEPTER)
chlorsulfuron (GLEAN)
chlorimuron (CLASSIC)
thiacarburon (HARMONY)

The enzyme activity graphs are given as Figures 3 to 8. In addition, homozygous lines of mutants 1 and 2 which were selected from generation $M_1BC_1S_2$, were also tested against the same group of herbicides and against representatives of the triazolopyrimidines and phenoxypyrimidines. The results are shown in Figures 9 to 16. The chemical structures of these herbicides are shown in Figure 2 of the accompanying drawings.

10. ASSESSMENT OF INHIBITION

From the enzyme assay data it is possible to derive, as a measure of the degree of inhibition the factor $ID_{50\%}$ for each mutant and for each herbicide. This factor is indicative of the herbicide concentration which gives 50% inhibition of the ALS activity in comparison with the wild-type control. It is also possible to derive the fold increase in resistance. These calculations are are given in Table 3 below.

TABLE 3

IMAZETHAPYR (heterozygous mutants

| Plant | ID50% µM | Fold Increase |
|---|---|---|
| Wild type | 1.5 | |
| Mutant 1 | >1000 | >660 |
| 2 | 230 | 153 |
| 3 | 2.5 | 2 |
| 5 | 125 | 83 |
| 6 | 60 | 40 |
| 7 | 4 | 3 |
| 8 | >1000 | >660 |
| 9 | 425 | 283 |
| 10 | 15 | 10 |

IMAZAPYR (heterozygous mutants)

| Plant | ID50% µM | Fold Increase |
|---|---|---|
| Wild type | 3 | |
| Mutant 1 | >1000 | >333 |
| 2 | 540 | 180 |
| 3 | 4 | 1 |
| 5 | 300 | 100 |
| 6 | 5 | 2 |
| 7 | 10 | 3 |
| 8 | >1000 | >333 |
| 9 | >1000 | >333 |
| 10 | 35 | 12 |

IMAZAQUIN (heterozygous mutants)

| Plant | ID50% µM | Fold Increase |
|---|---|---|
| Wild type | 0.6 | |
| Mutant 1 | 150 | 250 |
| 2 | 20 | 33 |
| 3 | 1.5 | 2 |
| 5 | 25 | 42 |
| 6 | 1.5 | 2 |
| 7 | 1 | 2 |
| 8 | 150 | 250 |
| 9 | 50 | 83 |
| 10 | 7 | 12 |

CHLORIMURON (heterozygous mutants)

| Plant | ID50% nM | Fold Increase |
|---|---|---|
| Wild type | 3.7 | |
| Mutant 1 | 5 | 1 |
| 2 | 10 | 3 |
| 3 | 15 | 4 |
| 5 | 17 | 5 |
| 6 | 15 | 4 |
| 7 | 5 | 1 |
| 8 | 10 | 3 |
| 9 | 10 | 3 |
| 10 | 17 | 5 |

CHLORSULFURON (heterozygous mutants)

| Plant | ID50% nM | Fold Increase |
|---|---|---|
| Wild type | 18.5 | |
| Mutant 1 | 65 | 4 |
| 2 | 65 | 4 |
| 3 | 35 | 2 |
| 5 | 40 | 2 |
| 6 | 65 | 4 |
| 7 | 8 | 1 |
| 8 | 75 | 4 |
| 9 | 50 | 3 |
| 10 | 35 | 2 |

THIACARBURON (heterozygous mutants)

| Plant | ID50% nM | Fold Increase |
|---|---|---|
| Wild type | 42.5 | |
| Mutant 1 | 55 | 1 |
| 2 | 320 | 8 |
| 3 | 85 | 2 |
| 5 | 340 | 8 |
| 6 | 45 | 1 |
| 7 | 25 | 1 |
| 8 | 40 | 1 |
| 9 | 45 | 1 |
| 10 | 390 | 9 |

Similar calculations were made for homozygous mutants 1 and 2 only, the results being given in Table 4 below.

TABLE 4

MUTANT 1 (homozygous)

| Herbicide | ID50% Wild | ID50% Mutant | Fold Increase |
|---|---|---|---|
| Imazapyr | 3μM | >1mM | >333 |
| Imazethapyr | 1.5μM | 750μM | 500 |
| Imazaquin | 0.6μM | 175μM | 292 |
| Chlorimuron | 3.7nM | 10nM | 2.7 |
| Chlorsulfuron | 18.5nM | 60nM | 3.2 |
| Thiacarburon | 42.5nM | 40nM | nil |

TABLE 4 (continued)

MUTANT 2 (homozygous)

| Herbicide | ID50% Wild | Mutant | Fold Increase |
|---|---|---|---|
| Imazapyr | 3μM | 675μM | 225 |
| Imazethapyr | 1.5μM | 400μM | 267 |
| Imazaquin | 0.6μM | 40μM | 66 |
| Chlorimuron | 3.7nM | 20nM | 5.4 |
| Chlorsulfuron | 18.5nM | 40nM | 2.2 |
| Thiacarburon | 42.5nM | 360nM | 8.5 |
| Triazolo-pyrimidine | 0.07μM | 0.12μM | 1.7 |
| Phenoxy-pyrimidine | 0.03mM | 0.45mM | 22.5 |

11. CROSS-RESISTANCE TESTING

The seeds used in this screen were populations of mixed tolerant and sensitive seed, segregating 1:1, derived from each of the heterogygous, tolerant mutants crossed with homozygous, sensitive UE95. All of the tolerant progeny are heterozygous for tolerance. Each of the mutants identified above were used except mutant 4. The controls were seed of self-pollinated UE95 which was pollinated in the same year as the tolerant lines.

One litre of compost was placed in each seed tray of dimensions 19cm x 11cm x 5cm deep and firmed down flat. Two furrows 1 cm deep were drawn in the surface of the compost in each tray and six seeds were sown in each furrow (total twelve seeds per tray). Some trays were sown with 24 seeds, in which case three furrows were made and 8 seeds sown per furrow.

In each test each tray contained either the seed of one mutant or of the UE95 control for each herbicide application rate and for the untreated control.

Five different types of ALS-inhibitor herbicides were tested on the mutants plus imazethapyr (PURSUIT) as a comparison. Four rates of each were applied; approximately 0.1x, 0.5x, 1x and 4x the estimated field rates for each herbicide. The rates are the same for mutant 3 to 10 but higher rates for chlorimuron (CLASSIC) and lower rates for chlorsulfuron (GLEAN) were applied to mutants 1 and 2.

The following herbicides, with their estimated field application rates were used:

| | |
|---|---|
| chlorimuron (CLASSIC) | 8 -13 g/Ha |
| chlorsulfuron (GLEAN) | 4 -26 g/HA |
| imazaquin (SCEPTER) | 100 -150 g/Ha |
| triazolopyrimidine | 10 - 30 g/Ha |
| phenoxypyrimidine | 100 - 200 g/Ha |
| imazethapyr (PURSUIT) | 70 - 140 g/Ha |

The rates of application used in the screening tests were as follows:

chlorimuron (CLASSIC) 4,20,40,160 g/Ha (mutants 1 and 2); and, 8,40,80,320 g/Ha (mutants 3 to 10);

chlorsulfuron (GLEAN) 5,25,50,200 g/Ha (mutants 1 and 2); and, 1,5,10,40 g/Ha (mutants 3 to 10);

imazaquin (SCEPTER) 30,150,300,1200 g/Ha;
triazolopyrimidine 5,25,50,200 g/Ha;
phenoxypyrimidine 10,50,100,400 g/Ha; and,
imazethapyr (PURSUIT) 30,150,300,1200 g/Ha

The triazolopyrimidine and phenoxypyrimidine were formulated in an adjuvant wetting agent known as JF5969 and diluted with water to make a final concentration of 10% JF5969. The remaining compounds were diluted with water only.

The spray jet and parameters were as decribed above under "SCREENING". After spraying, the seeds were covered with 0.5 litre of compost and firmed down flat (2.5cm covering) and grown under the conditions described under "SCREENING" above.

Visual assessments were made of the plants after four weeks growth. Each plant was scored on a scale of zero to 5 and the height measured from soil surface to the tallest leaf tip. The scoring scale was as follows:

0 - 0 to 10% damage (little or no herbicidal effect)
1 - 11 to 25% damage
2 - 26 to 50% damage
3 - 51 to 85% damage
4 - 81 to 95% damage
5 - 96 to 100% damage (complete death of the plant)

In carrying out these assessments, plants which were obviously of the sensitive phenotype were ignored (approximately 50% of the plants).

Plants scoring 0, 1 or 2 were recorded and potted on into larger pots and grown to maturity.

The results are shown in Figures 17 to 22. Figure 23 shows a comparison of the results obtained as between heterozygous and homozygous plants.

## 12. INTERPRETATION OF THE RESULTS

The data generated for the heterozygotes by the enzyme assays and the dose response curves on the living plants, do not correlate precisely. To those skilled in the art, this will not be particularly unexpected. However, there is a general correlation in the sense that the general level of resistance shown in enzyme assays tends to be reflected in the glasshouse studies. Of course, the criteria for deciding what constitutes a useful mutant varies according to the herbicide, or spectrum of herbicides and the rate of application of the herbicide with which one is dealing and the relatively rich diversity in the spectrum of cross-resistance displayed by the mutants of this invention is seen as an advantage rather than an undesirable variation, allowing, as it does, selection of a mutant for the circumstances of intended use. For example, although a particular mutant may display relatively low resistance compared with the others against a particular herbicide this may make it eminently useful for providing plants which are intended to tolerate only small amounts of herbicide, for example, to resist a "carry-over" effect.

With this in mind, it is therefore possible to categorise the mutants on the basis of the enzyme assays and the glasshouse tests into "strong", "intermediate", "weak" and "zero" resistance groups for each herbicide. This classification is summarised in Table 5 below.

TABLE 5

| IMAZETHAPYR | MUTANT NUMBER | |
|---|---|---|
| | Enzyme Assay | Dose Response |
| Strong | 1,8,9 | 1,5,8,9 |
| Intermediate | 2,5,10 | 2,6,10 |
| Weak | 3,6,7, | 7,3 |
| Zero | - | - |
| IMAZAPYR | Enzyme Assay | Dose Response |
| Strong | 1,2,5,8,9,10 | no data |
| Intermediate | 6,7 | no data |
| Weak | 3 | no data |
| Zero | - | no data |
| IMAZAQUIN | Enzyme Assay | Dose Response |
| Strong | 1,8,9 | 1,2,8,9 |
| Intermediate | 2,5 | 5 |
| Weak | 3,6,7,10 | 3,6,10 |
| Zero | | 7 |
| CHLORSULFURON | Enzyme Assay | Dose Response |
| Strong | - | - |
| Intermediate | -, | 3 |
| Weak | 1,2,3,5,6,8,9,10 | 5,7,9,10 |
| Zero | 7 | 1,2,6,8,9,10 |
| CHLORIMURON | Enzyme Assay | Dose Response |
| Strong | - | - |
| Intermediate | 10 | - |
| Weak | 1,2,3,5,6,7,9 | 3,10 |
| Zero | 8 | 1,2,5,6,7,8,9 |
| THIACARBURON | Enzyme Assay | Dose Response |
| Strong | - | no data |
| Intermediate | 2,5,10 | no data |
| Weak | 1,3,6,8,9 | no data |
| Zero | 7 | no data |

TABLE 5 (continued)

| TRIAZOLOPYRIMIDINE | | |
|---|---|---|
| | Enzyme Assay | Dose Response |
| Strong | no data | 3 |
| Intermediate | no data | 1,2 |
| Weak | no data | 7,8,9 |
| Zero | no data | 5,6,10 |
| PHENOXYPYRIMIDINE | | |
| | Enzyme Assay | Dose Response |
| Strong | no data | - |
| Intermediate | no data | 2,5,7 |
| Weak | no data | 3,8,9,10 |
| | no data | 1,6 |

13. PLANT BREEDING

The mutated lines of the present invention can be used in common with various second parent lines to produce herbicide resistant hybrids.

Material from the homozygous lines may be entered into a breeding programme involving further outcrossing, selfing, visual selection and herbicide screening in order to produce a range of new herbicide-tolerant hybrid seed.

The herbicide resistance trait can be transferred to new lines by the described mutation breeding approach or by conventional breeding practices, using selection for herbicide resistance as described hereinabove. Biochemical and molecular screening techniques are also available to those skilled in the art to aid the process. The use of genetic engineering techniques are readily conceivable to isolate and transfer the resistance gene.

The objective of a breeding preogramme may simply be the beneficial transfer of herbicide resistance or may be more complex, involving concurrent improvement of agronomic performance.

**Claims**

1. A method of producing mutant Zea mays possessing resistance to a normally lethal concentration of a chosen herbicide, comprising subjecting isolated pollen of Zea mays to the action of a chemical mutagen, recovering mutagenised pollen, pollinating a female parent therewith, harvesting seed from the mature plant, growing the seeds in the presence of selection pressure of the chosen herbicide or a second herbicide having the same mode of action as the chosen herbicide and recovering plants and their progeny which are resistant to the applied selection pressure.

2. A method as claimed in claim 1 in which the chemical mutagen is ethyl methanesulphonate.

3. A method as claimed in claim 1 in which the herbicide utilised for applying selection pressure is an inhibitor of acetolactate synthase.

4. A method as claimed in claim 3 in which the said herbicide is imazethapyr.

5. A method as claimed in any preceding claims, in which the concentration of herbicide used for selection pressure is insufficient to inhibit germination but sufficient to inhibit growth of sensitive plants.

6. A method as claimed in any preceding claim in which the selection pressure is applied by application of the herbicide to the seeds pre-emergence.

## Patentansprüche

1. Verfahren zur Erzeugung von Zea-mays-Mutanten, die resistent gegen eine normalerweise tödliche Konzentration eines ausgewählten Herbicids sind, bei dem isolierter Pollen von Zea mays der Einwirkung eines chemischen Mutagens ausgesetzt wird, der mutierte Pollen gewonnen wird, ein weiblicher Elternteil damit bestäubt wird, die Samen der reifen Pflanze geerntet werden, die Samen unter dem Selektionsdruck des ausgewählten Herbicids oder eines zweiten Herbicids, das den gleichen Wirkungsmechanismus wie das ausgewählte Herbicid aufweist, gezüchtet und die Pflanzen und ihre Nachkommen gewonnen werden, die gegen den ausgeübten Selektionsdruck resistent sind.

2. Verfahren nach Anspruch 1, wobei das chemische Mutagen Ethylmethansulfonat ist.

3. Verfahren nach Anspruch 1, wobei das zur Ausübung des Selektionsdrucks verwendete Herbicid ein Inhibitor von Acetolactat-Synthase ist.

4. Verfahren nach Anspruch 3, wobei das Herbicid Imazethapyr ist.

5. Verfahren nach einem der vorhergehenden Ansprüche, wobei die Konzentration des für den Selektionsdruck verwendeten Herbicids nicht ausreicht, um die Keimung zu verhindern, aber ausreichend ist, um das Wachstum von empfindlichen Pflanzen zu verhindern.

6. Verfahren nach einem der vorhergehenden Ansprüche, wobei der Selektionsdruck durch Vorlauf-Aufbringung des Herbicids auf die Samen ausgeübt wird.

## Revendications

1. Procédé de production de Zea mays mutant résistant aux concentrations ordinairement létales d'un herbicide sélectionné, comprenant la soumission de pollen isolé de Zea mays à l'action d'un mutagène chimique, la collecte du pollen mutant, la pollinisation d'une plante femelle parentale avec ce dernier, la récolte des semences à partir de la plante mûre, la croissance des semences sous pression sélective de l'herbicide sélectionné ou d'un second herbicide ayant un mode d'action identique à l'herbicide sélectionné et la collecte des plantes et de leur descendance qui présentent une résistance lorsqu'elles sont soumises à la pression sélective.

2. Procédé selon la revendication 1, dans lequel le mutagène chimique est l'éthylméthanesulfonate.

3. Procédé selon la revendication 1 dans lequel l'herbicide utilisé pour appliquer une pression sélective est un inhibiteur de l'acétolactate synthétase.

4. Procédé selon la revendication 3 dans lequel ledit herbicide est l'imazéthapyr.

5. Procédé selon l'une quelconque des revendications précédentes, dans lequel la concentration de l'herbicide utilisé pour appliquer la pression sélective est insuffisante pour inhiber la germination mais suffisante pour inhiber la croissance des plantes sensibles.

6. Procédé selon l'une quelconque des revendications précédentes dans lequel la pression sélective se fait par application de l'herbicide aux semences avant germination.

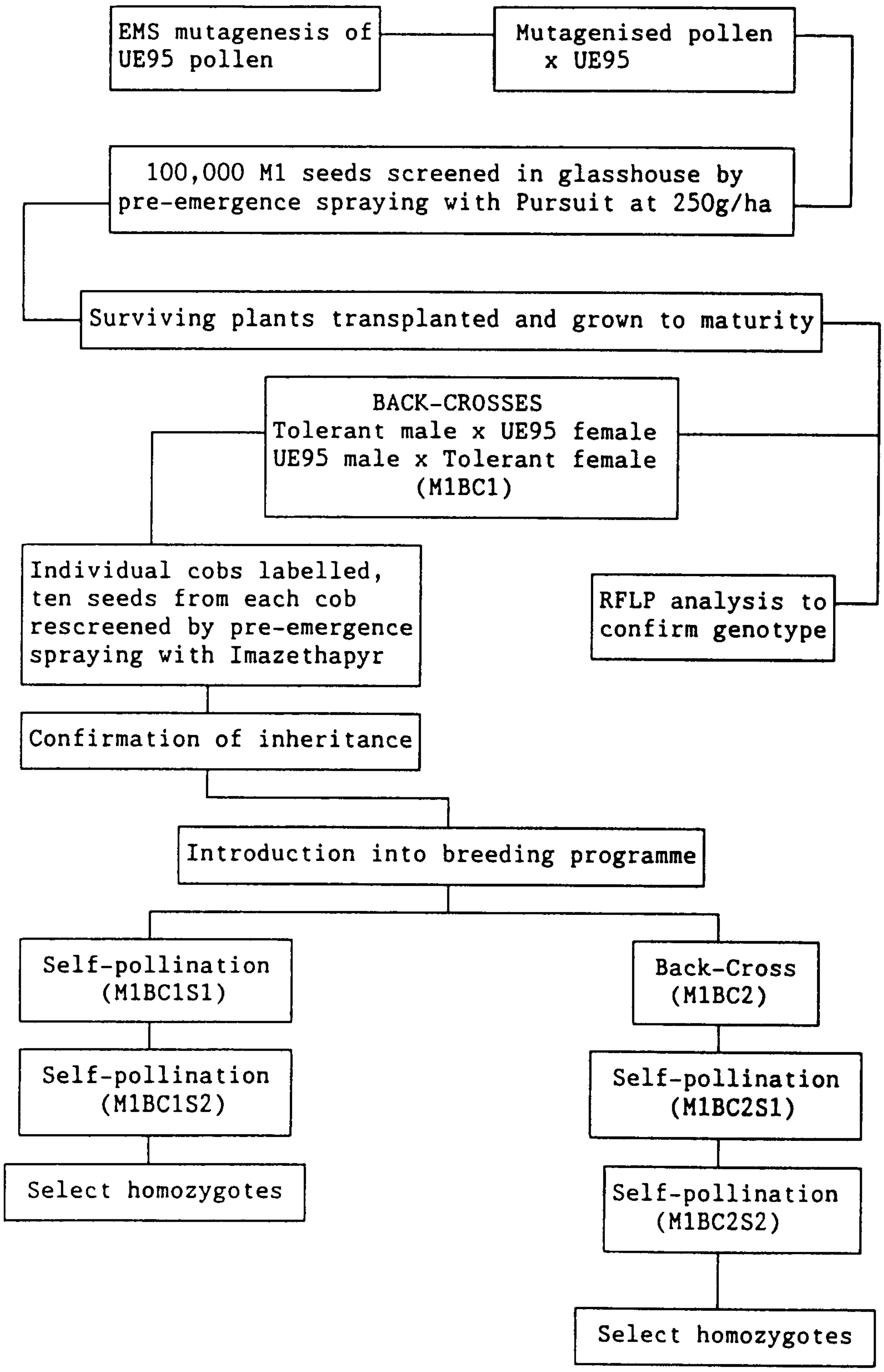
EMS mutagenesis of UE95 pollen
Mutagenised pollen x UE95
100,000 M1 seeds screened in glasshouse by pre-emergence spraying with Pursuit at 250g/ha
Surviving plants transplanted and grown to maturity
BACK-CROSSES
Tolerant male x UE95 female
UE95 male x Tolerant female
(M1BC1)
Individual cobs labelled, ten seeds from each cob rescreened by pre-emergence spraying with Imazethapyr
RFLP analysis to confirm genotype
Confirmation of inheritance
Introduction into breeding programme
Self-pollination (M1BC1S1)
Self-pollination (M1BC1S2)
Select homozygotes
Back-Cross (M1BC2)
Self-pollination (M1BC2S1)
Self-pollination (M1BC2S2)
Select homozygotes

FIG. 1.

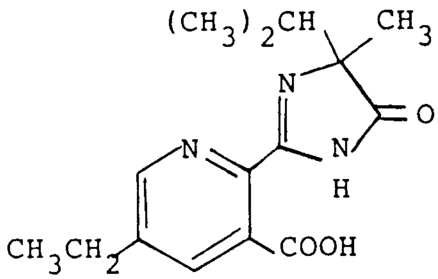

IMAZETHAPYR

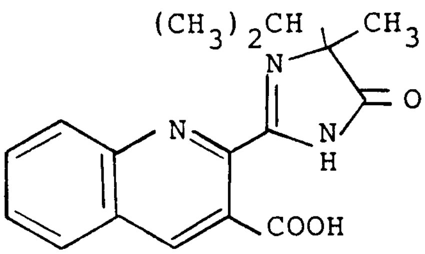

IMAZAQUIN

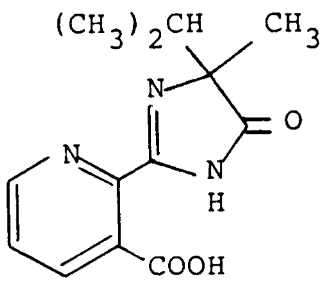

IMAZAPYR

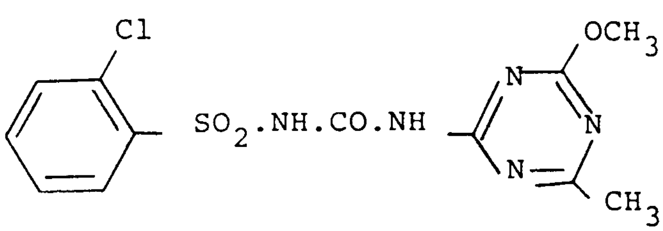

CHLORSULFURON

FIG. 2.

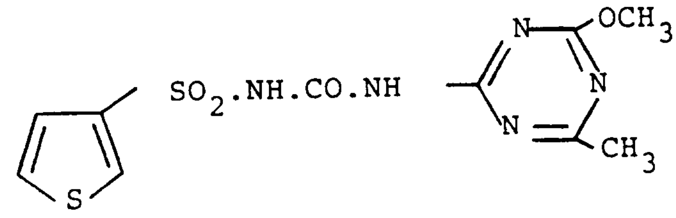

THIACARBURON

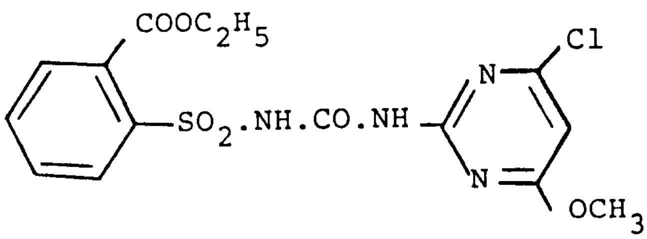

CHLORIMURON

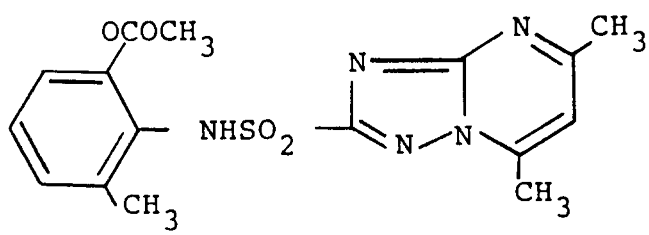

TRIAZOLO-
PYRIMIDINE

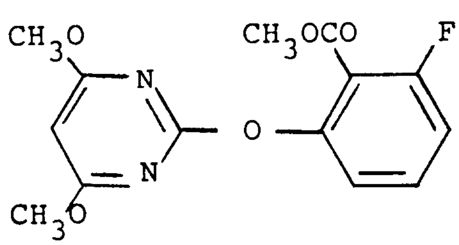

PHENOXYPYRIMIDINE

FIG. 2 Continued

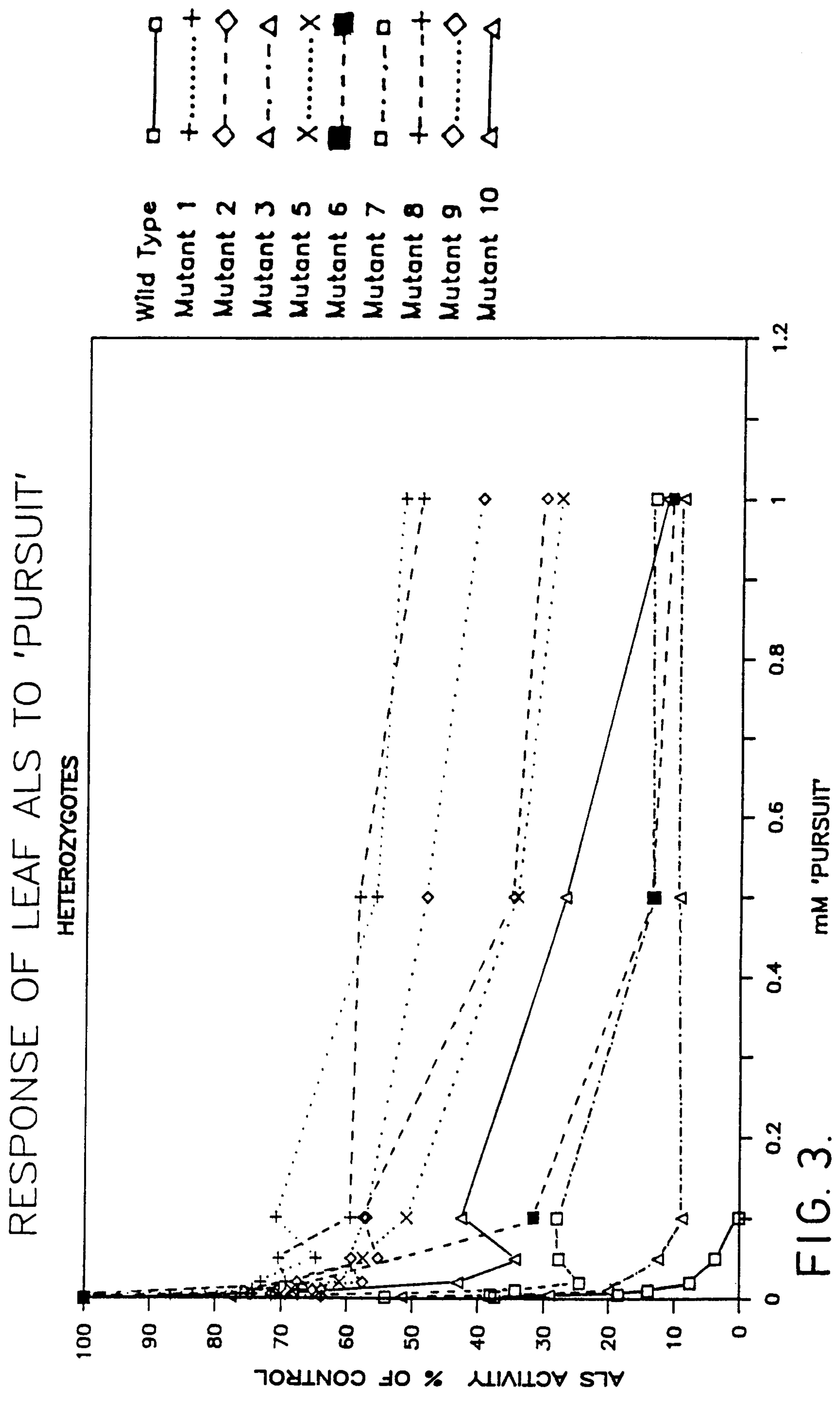
RESPONSE OF LEAF ALS TO 'PURSUIT'
HETEROZYGOTES
ALS ACTIVITY % OF CONTROL
100
90
80
70
60
50
40
30
20
10
0
0
0.2
0.4
0.6
0.8
1
1.2
mM 'PURSUIT'
Wild Type
Mutant 1
Mutant 2
Mutant 3
Mutant 5
Mutant 6
Mutant 7
Mutant 8
Mutant 9
Mutant 10

FIG. 3.

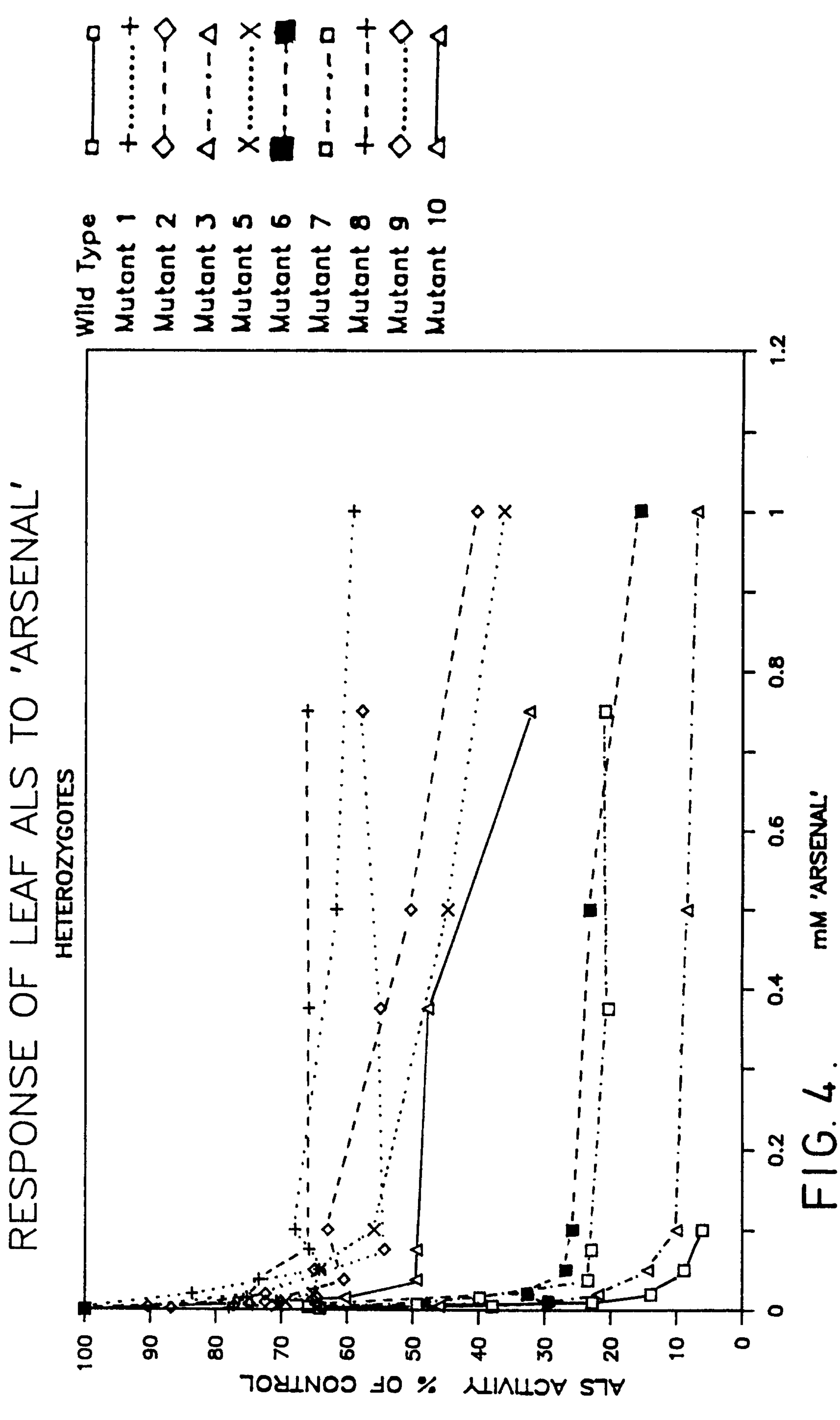
RESPONSE OF LEAF ALS TO 'ARSENAL'
HETEROZYGOTES
ALS ACTIVITY % OF CONTROL
mM 'ARSENAL'
0
0.2
0.4
0.6
0.8
1
1.2
100
90
80
70
60
50
40
30
20
10
0
Wild Type
Mutant 1
Mutant 2
Mutant 3
Mutant 5
Mutant 6
Mutant 7
Mutant 8
Mutant 9
Mutant 10

FIG. 4.

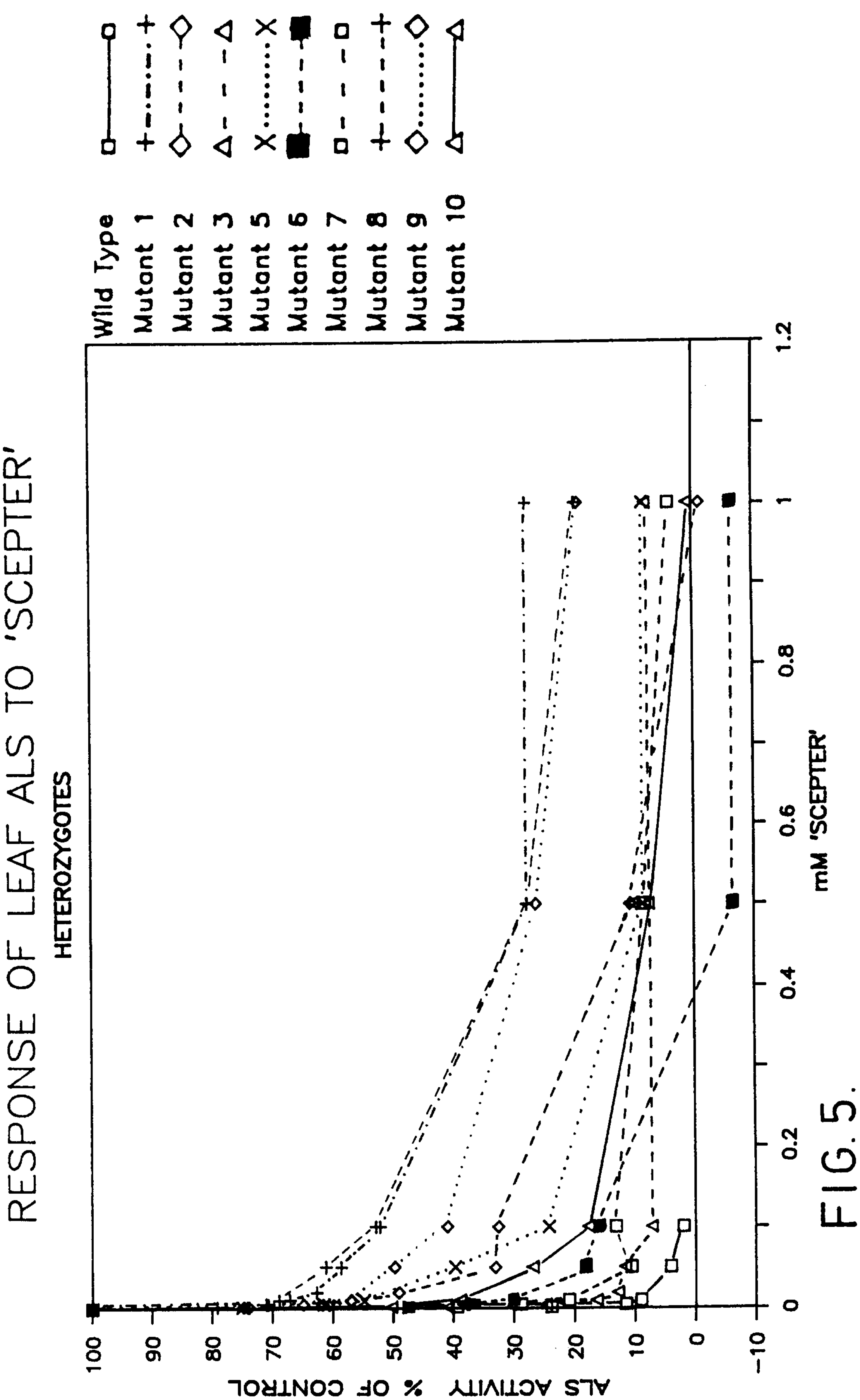
RESPONSE OF LEAF ALS TO 'SCEPTER'
HETEROZYGOTES
Wild Type
Mutant 1
Mutant 2
Mutant 3
Mutant 5
Mutant 6
Mutant 7
Mutant 8
Mutant 9
Mutant 10
ALS ACTIVITY % OF CONTROL
100
90
80
70
60
50
40
30
20
10
0
-10
0
0.2
0.4
0.6
0.8
1
1.2
mM 'SCEPTER'

FIG. 5.

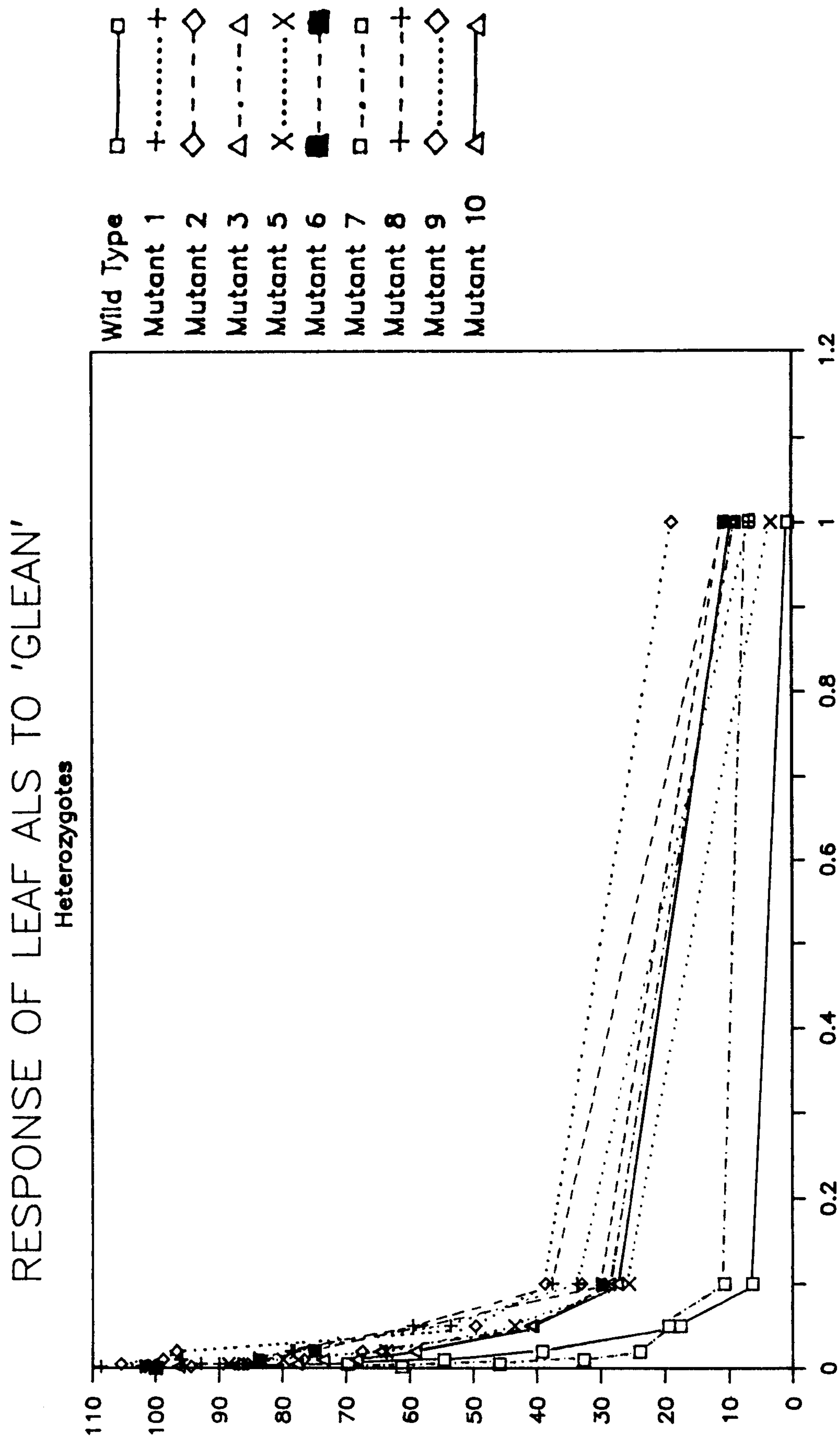
RESPONSE OF LEAF ALS TO 'GLEAN'
Heterozygotes
ALS ACTIVITY % OF CONTROL
110
100
90
80
70
60
50
40
30
20
10
0
0
0.2
0.4
0.6
0.8
1
1.2
uM 'GLEAN'
Wild Type
Mutant 1
Mutant 2
Mutant 3
Mutant 5
Mutant 6
Mutant 7
Mutant 8
Mutant 9
Mutant 10

FIG. 6.

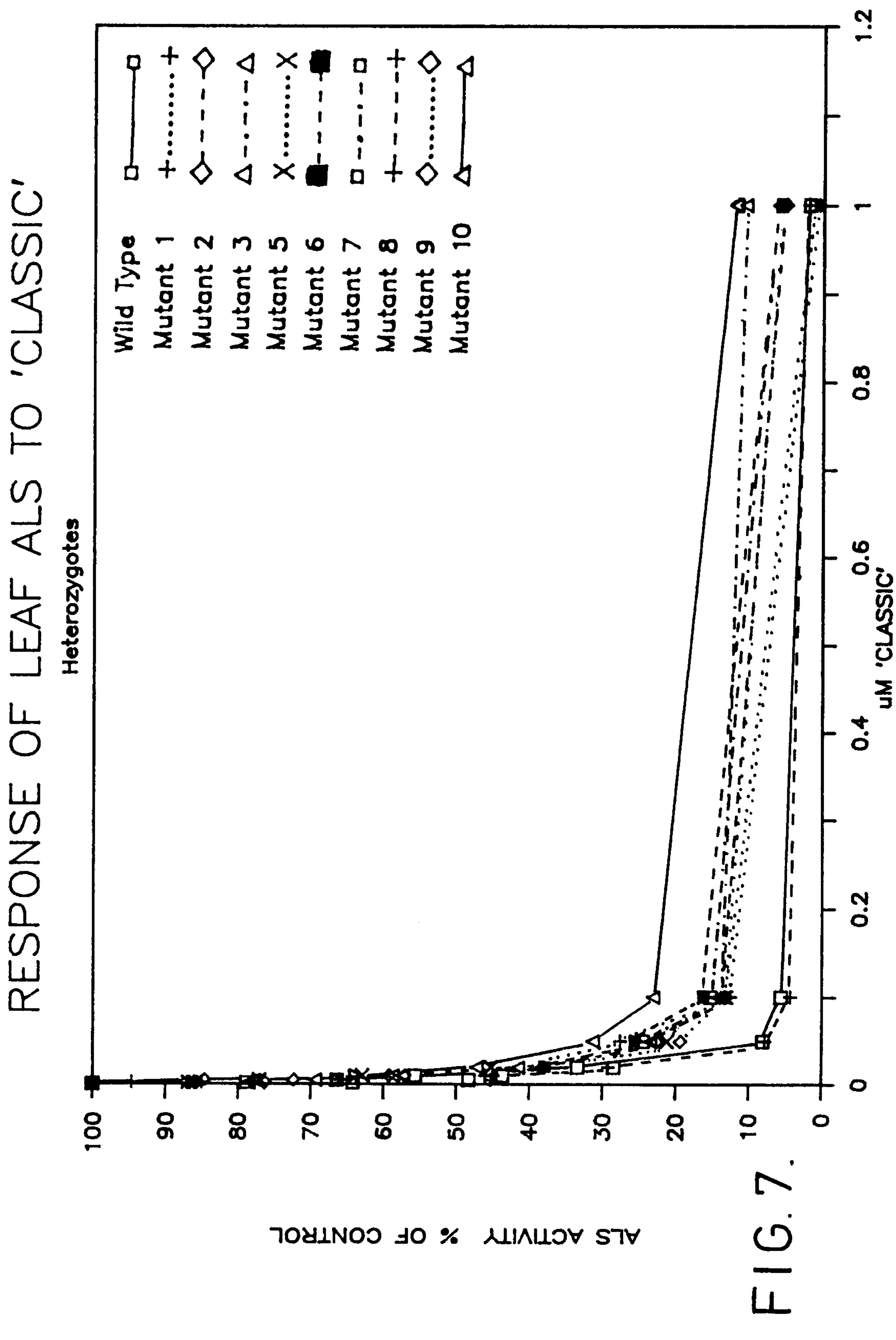
RESPONSE OF LEAF ALS TO 'CLASSIC'
Heterozygotes
Wild Type
Mutant 1
Mutant 2
Mutant 3
Mutant 5
Mutant 6
Mutant 7
Mutant 8
Mutant 9
Mutant 10
ALS ACTIVITY % OF CONTROL
uM 'CLASSIC'
100
90
80
70
60
50
40
30
20
10
0
0
0.2
0.4
0.6
0.8
1
1.2

FIG. 7.

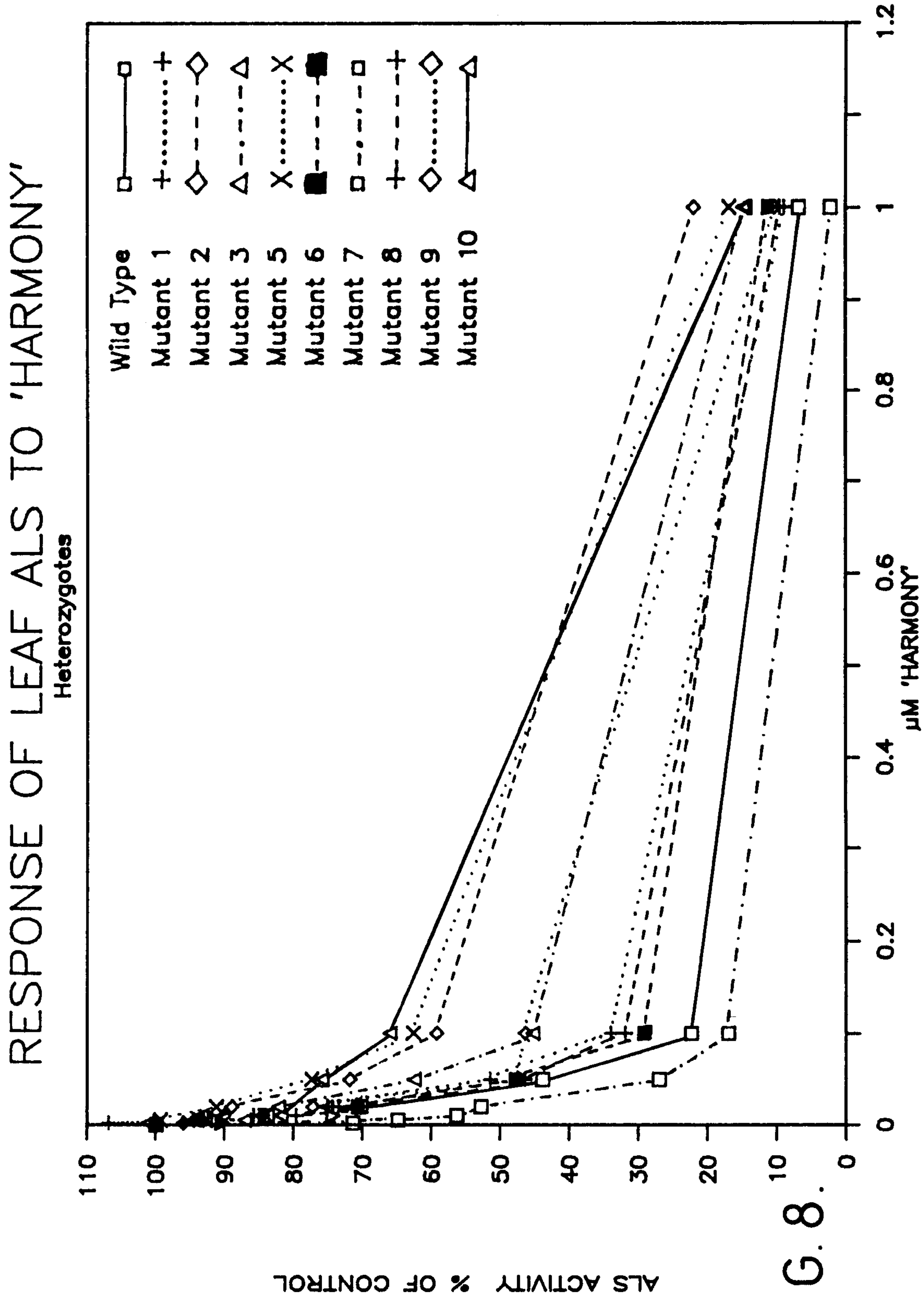
RESPONSE OF LEAF ALS TO 'HARMONY'
Heterozygotes
Wild Type
Mutant 1
Mutant 2
Mutant 3
Mutant 5
Mutant 6
Mutant 7
Mutant 8
Mutant 9
Mutant 10
ALS ACTIVITY % OF CONTROL
110
100
90
80
70
60
50
40
30
20
10
0
0
0.2
0.4
0.6
0.8
1
1.2
µM 'HARMONY'

FIG. 8.

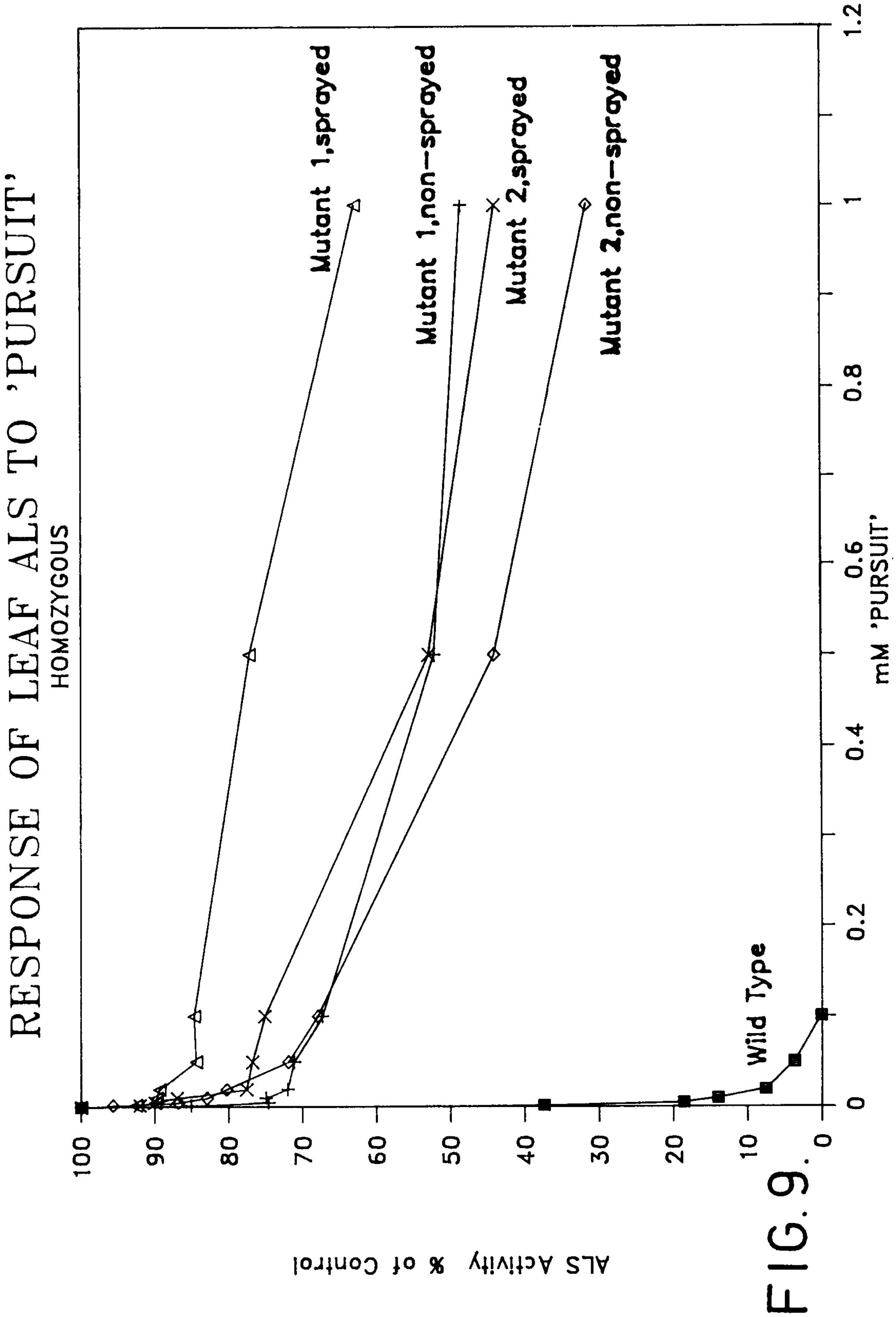
RESPONSE OF LEAF ALS TO 'PURSUIT'
HOMOZYGOUS
Mutant 1,sprayed
Mutant 1,non-sprayed
Mutant 2,sprayed
Mutant 2,non-sprayed
Wild Type
ALS Activity % of Control
mM 'PURSUIT'
100
90
80
70
60
50
40
30
20
10
0
0
0.2
0.4
0.6
0.8
1
1.2

FIG. 9.

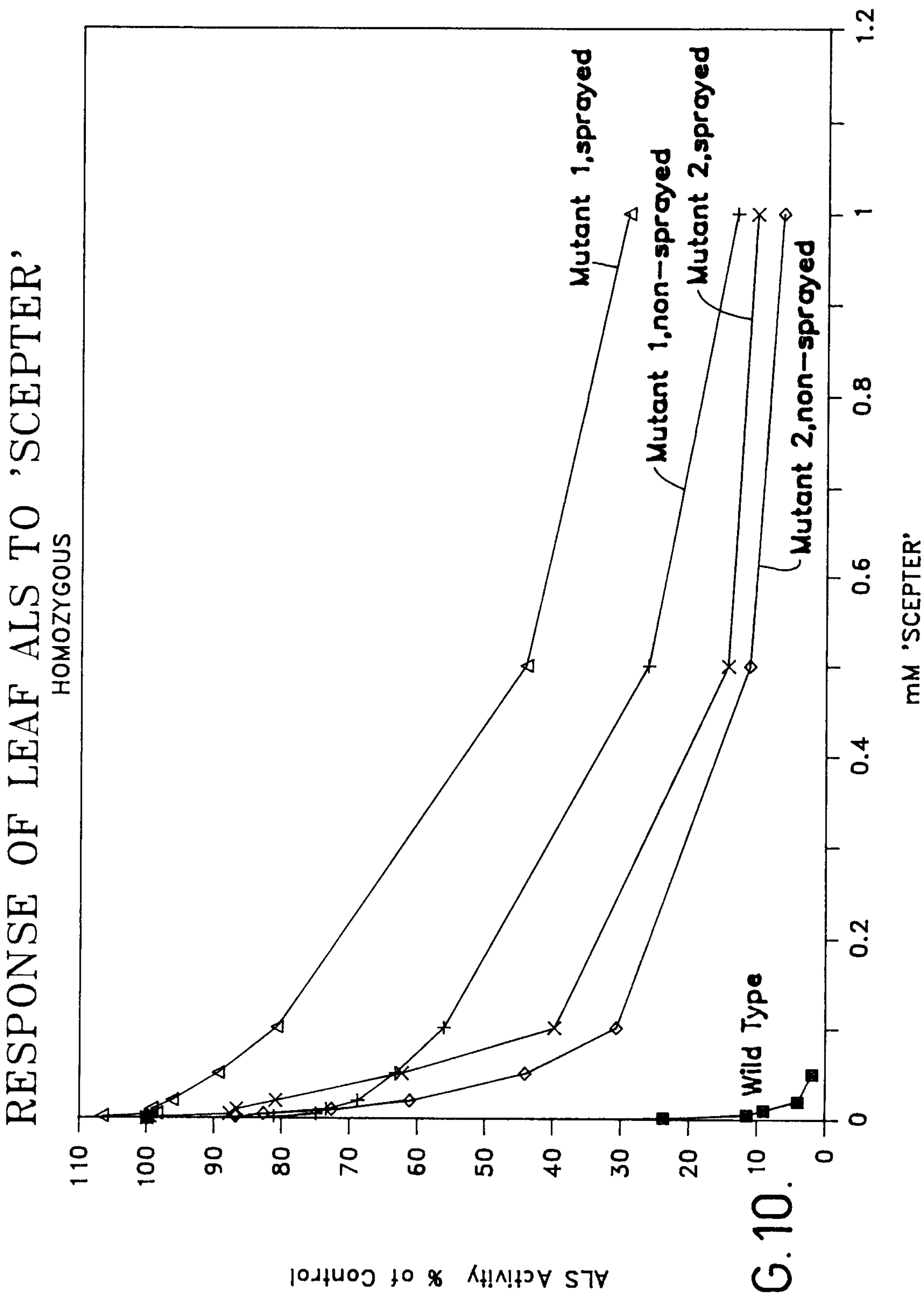
RESPONSE OF LEAF ALS TO 'SCEPTER'
HOMOZYGOUS
ALS Activity % of Control
110
100
90
80
70
60
50
40
30
20
10
0
mM 'SCEPTER'
0
0.2
0.4
0.6
0.8
1
1.2
Mutant 1,sprayed
Mutant 1,non-sprayed
Mutant 2,sprayed
Mutant 2,non-sprayed
Wild Type

FIG.10.

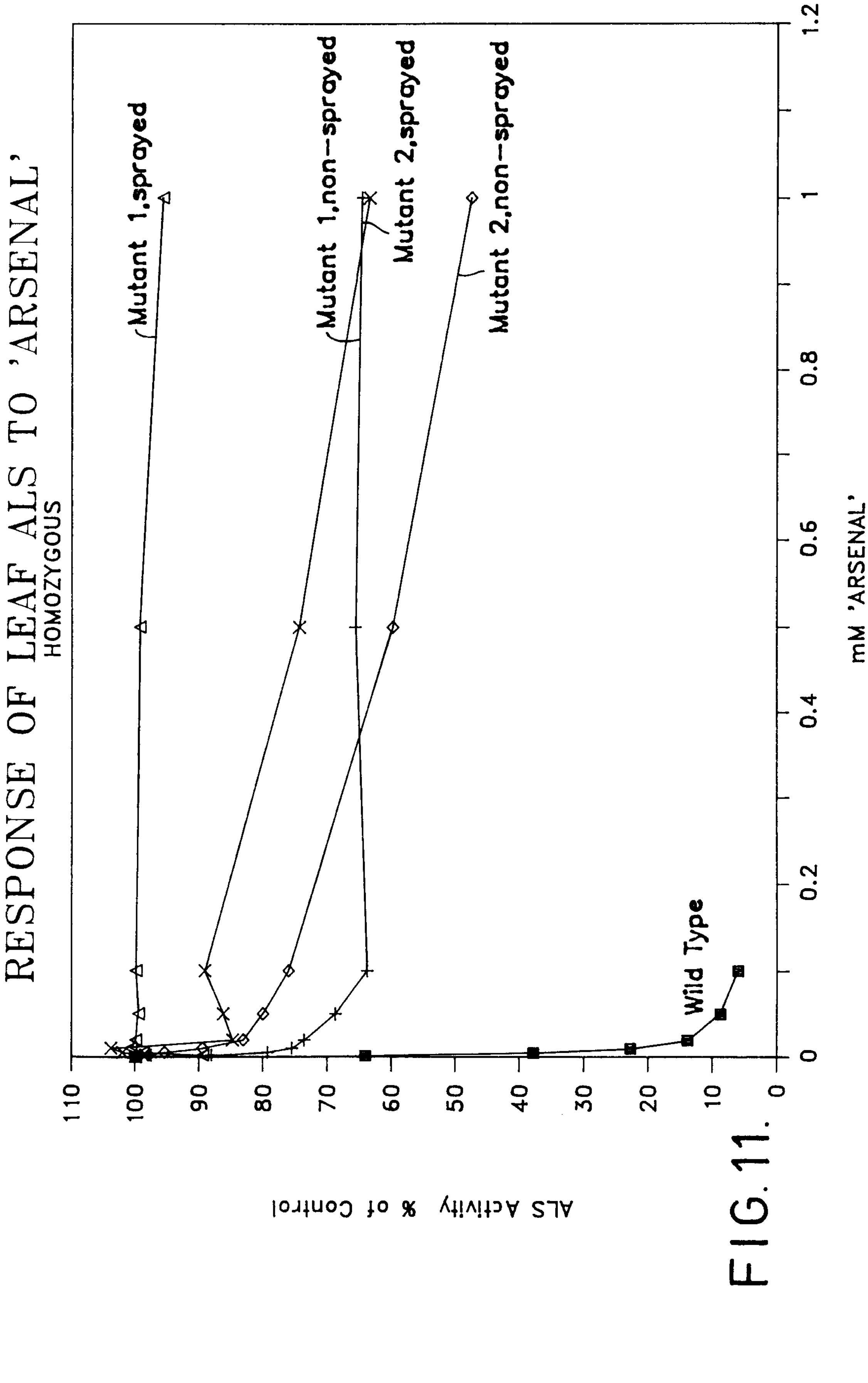
RESPONSE OF LEAF ALS TO 'ARSENAL'
HOMOZYGOUS
Mutant 1,sprayed
Mutant 1,non-sprayed
Mutant 2,sprayed
Mutant 2,non-sprayed
Wild Type
ALS Activity % of Control
mM 'ARSENAL'
110
100
90
80
70
60
50
40
30
20
10
0
0
0.2
0.4
0.6
0.8
1
1.2

FIG.11.

FIG. 12. RESPONSE OF LEAF ALS TO 'GLEAN'

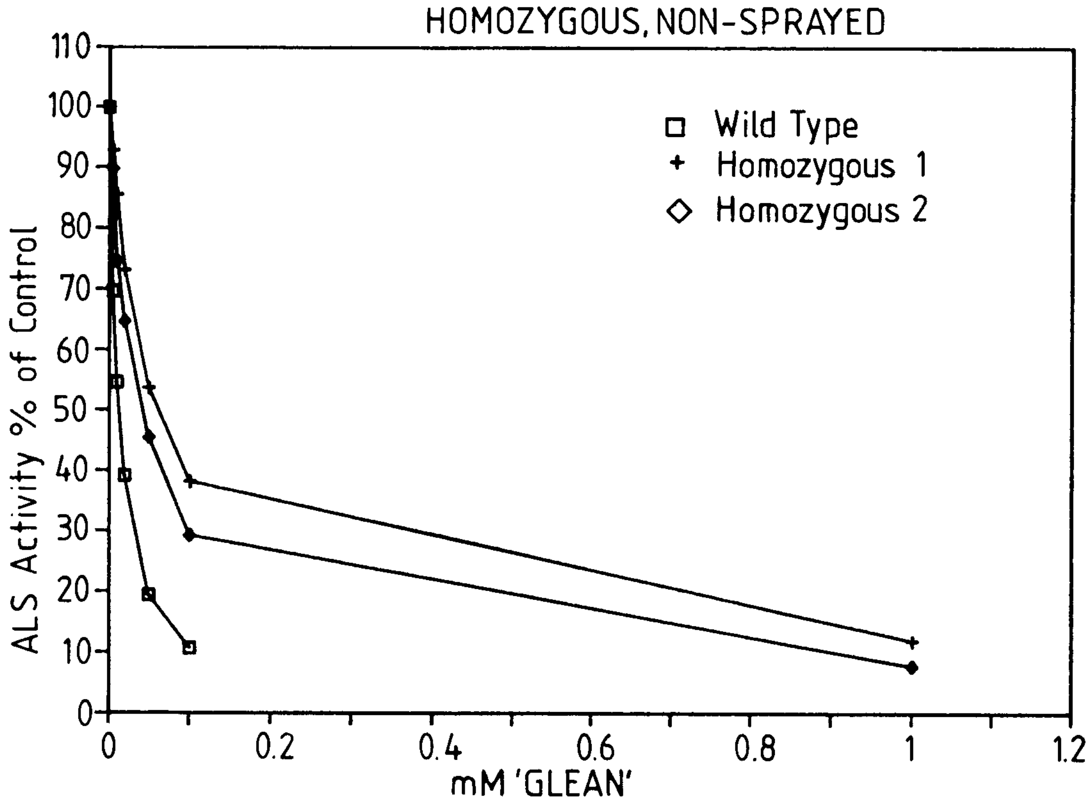


FIG. 13. RESPONSE OF LEAF ALS TO 'CLASSIC'

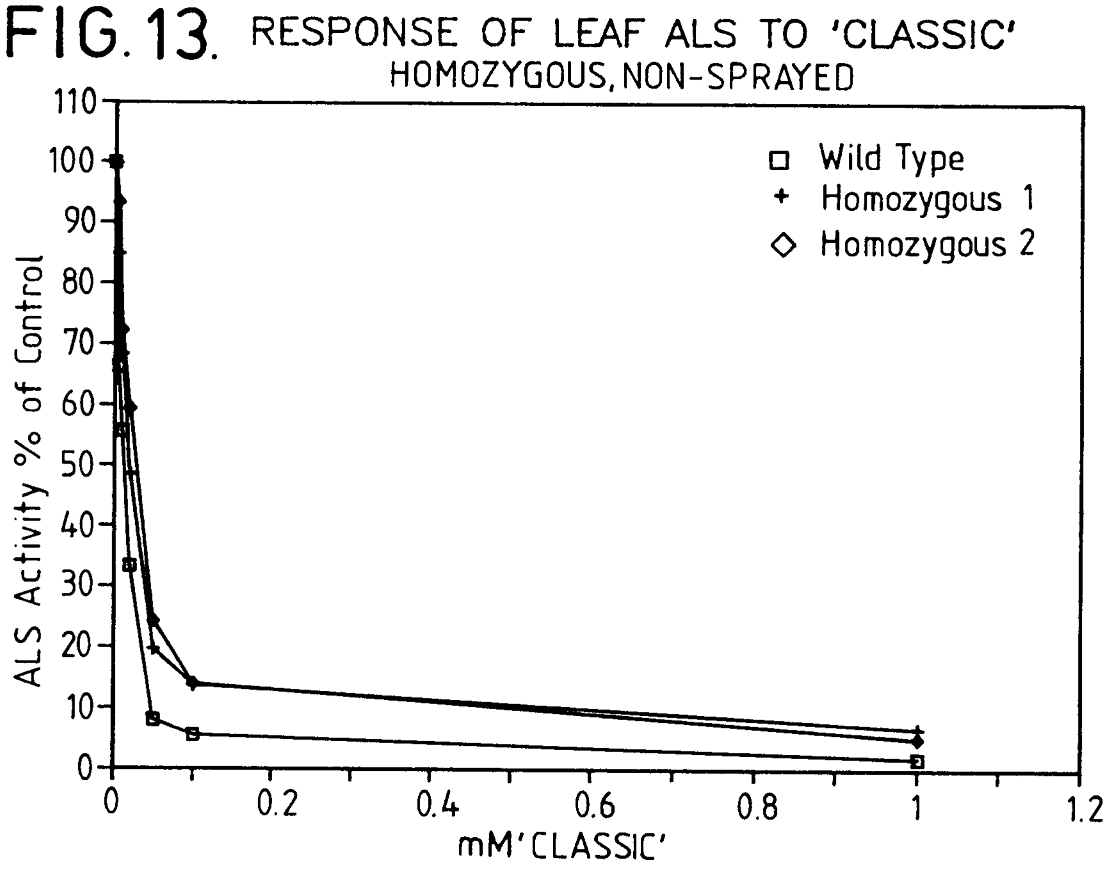

FIG. 14.

RESPONSE OF LEAF ALS TO 'HARMONY'

HOMOZYGOUS, NON-SPRAYED

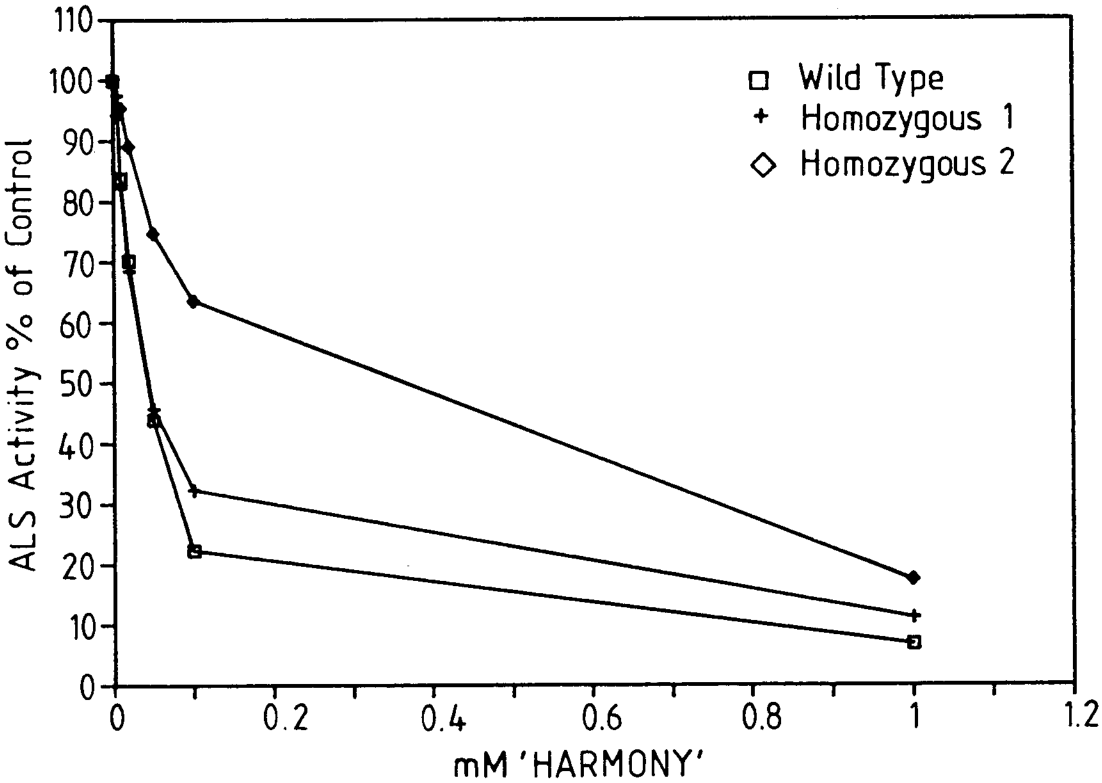

FIG. 15. RESPONSE OF ALS TO A PHENOXYPYRIMIDINE

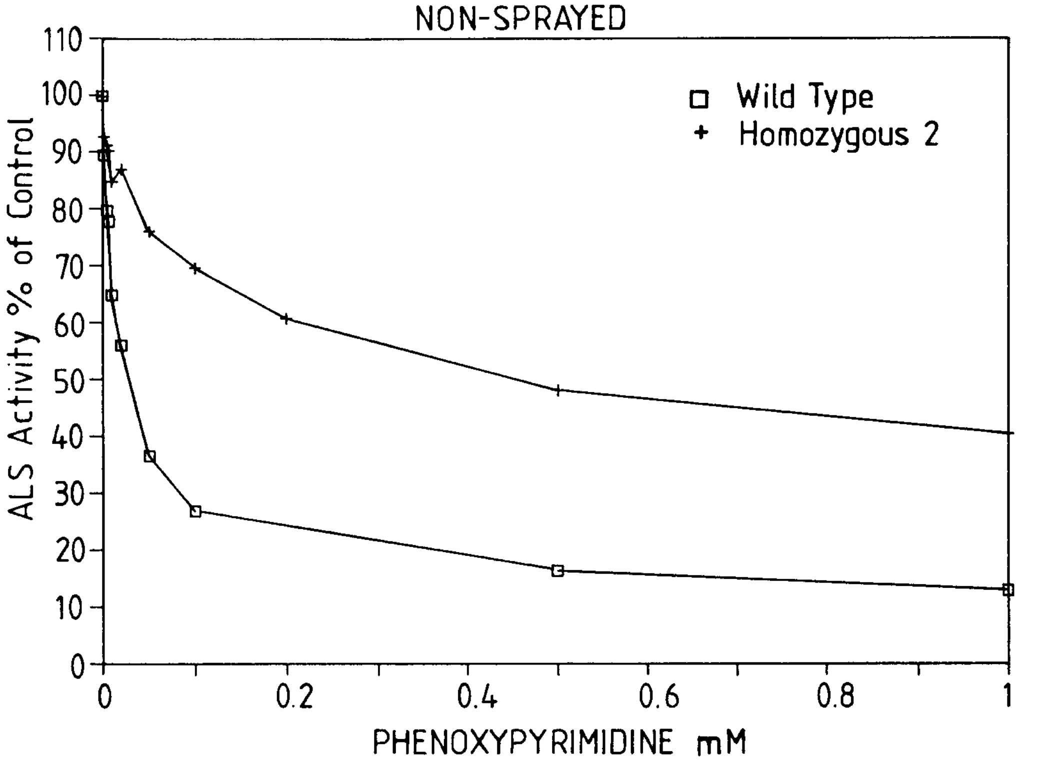


FIG. 16. RESPONSE OF ALS TO A TRIAZOLOPYRIMIDINE

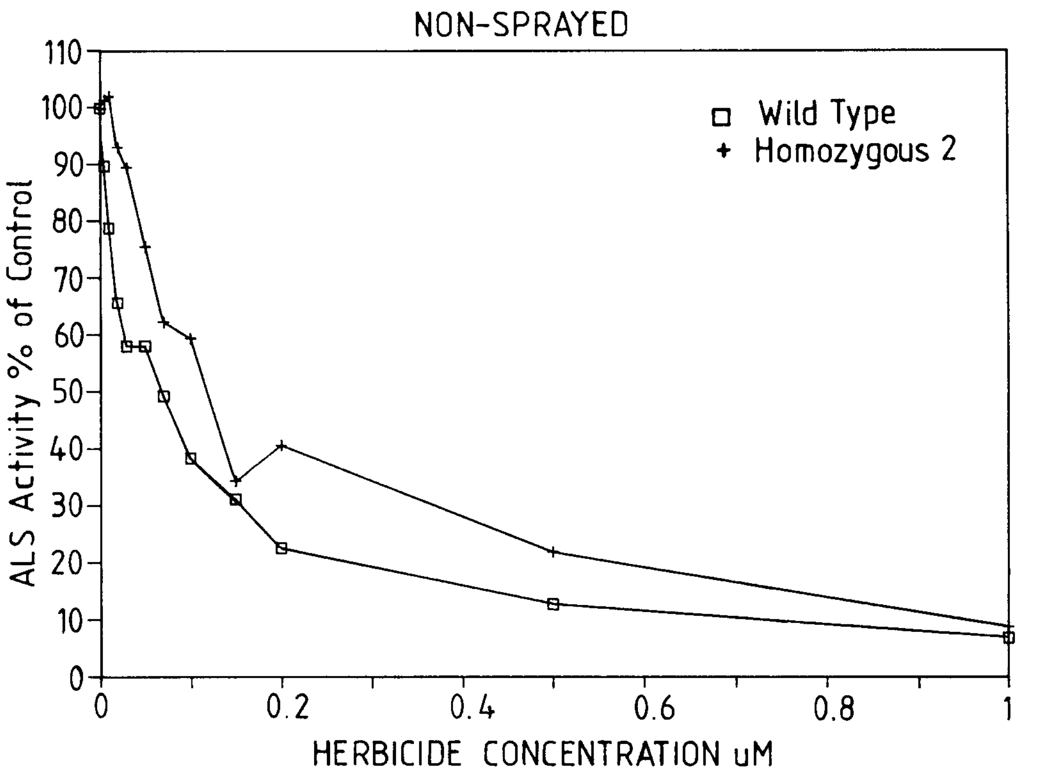

FIG. 17.

PURSUIT DOSE RESPONSE

HETEROZYGOTES PLANT HEIGHT 28 DAT

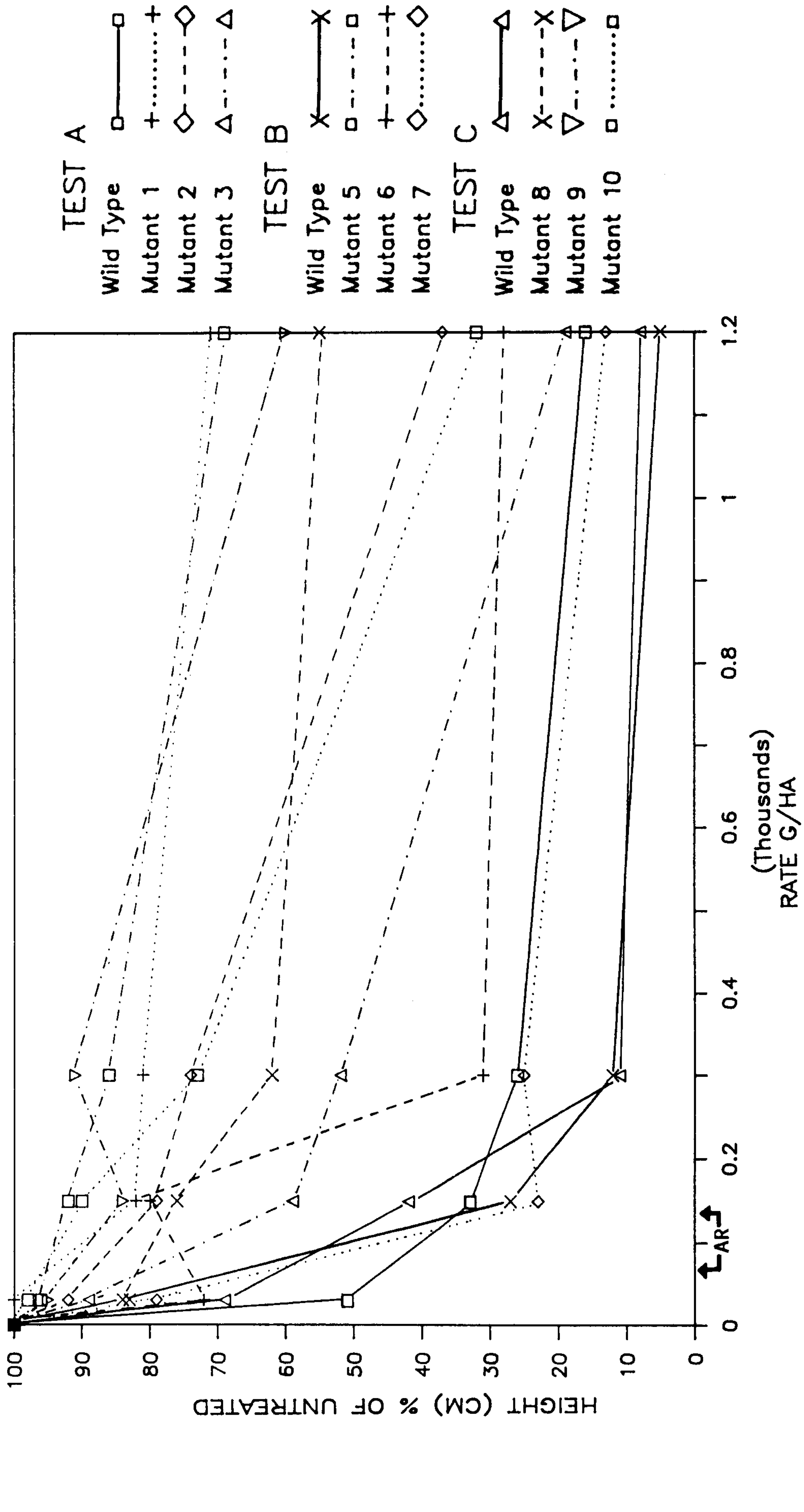

FIG. 18.

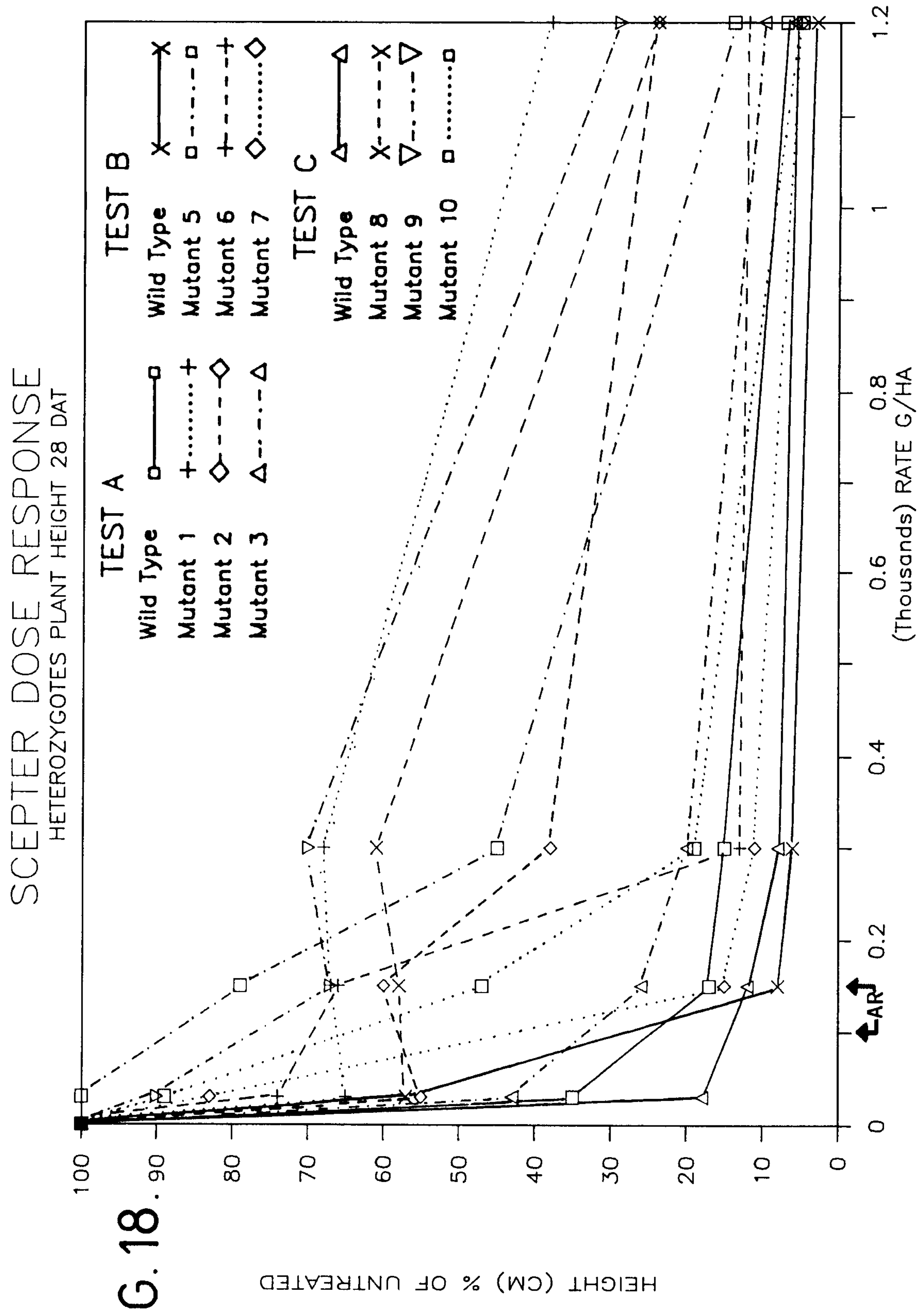
SCEPTER DOSE RESPONSE
HETEROZYGOTES PLANT HEIGHT 28 DAT
TEST A
Wild Type
Mutant 1
Mutant 2
Mutant 3
TEST B
Wild Type
Mutant 5
Mutant 6
Mutant 7
TEST C
Wild Type
Mutant 8
Mutant 9
Mutant 10
HEIGHT (CM) % OF UNTREATED
(Thousands) RATE G/HA
AR
100
90
80
70
60
50
40
30
20
10
0
0
0.2
0.4
0.6
0.8
1
1.2

FIG. 19.

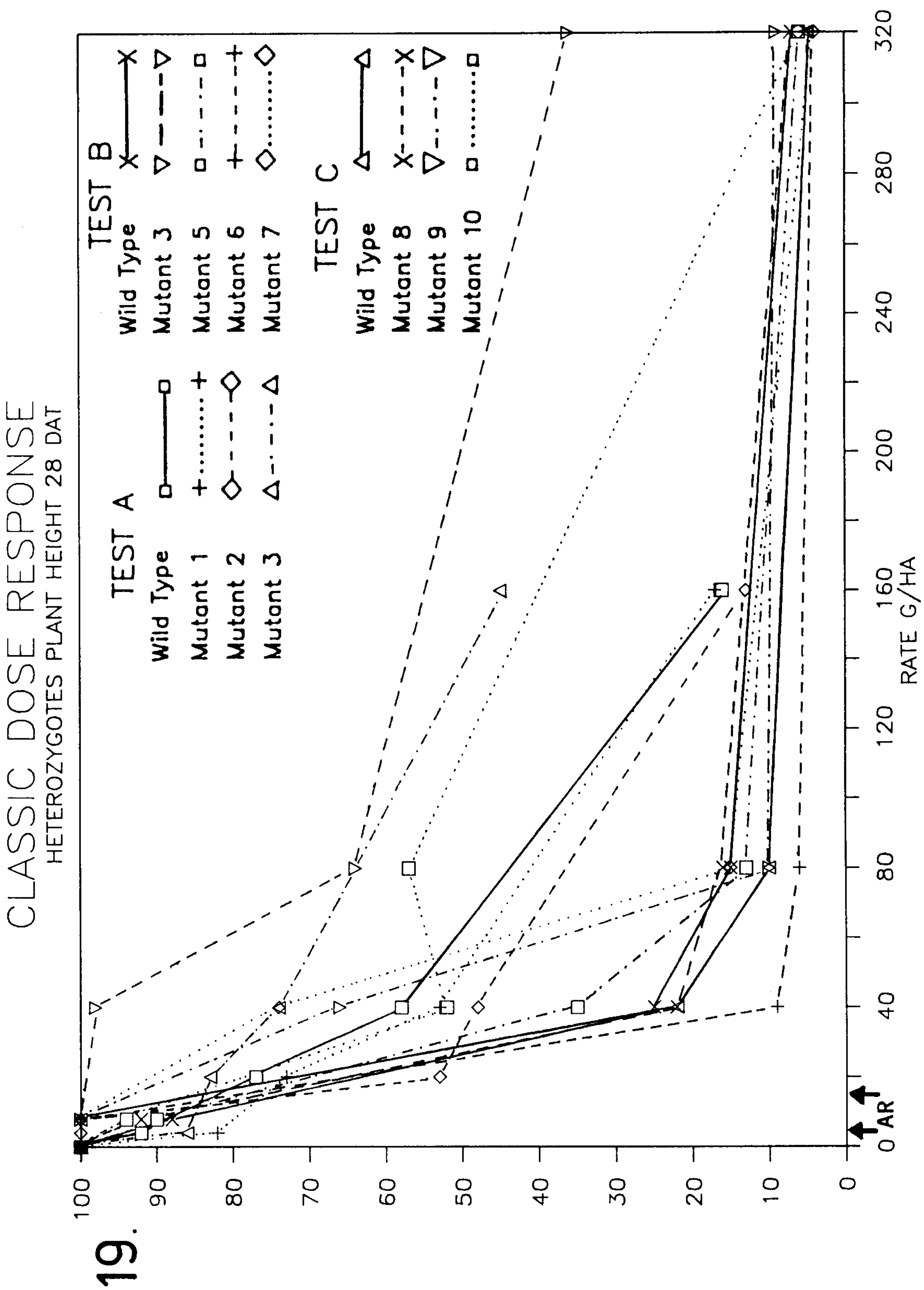
CLASSIC DOSE RESPONSE
HETEROZYGOTES PLANT HEIGHT 28 DAT
TEST A
Wild Type
Mutant 1
Mutant 2
Mutant 3
TEST B
Wild Type
Mutant 3
Mutant 5
Mutant 6
Mutant 7
TEST C
Wild Type
Mutant 8
Mutant 9
Mutant 10
HEIGHT (CM) % OF UNTREATED
100
90
80
70
60
50
40
30
20
10
0
0
AR
40
80
120
160
200
240
280
320
RATE G/HA

FIG. 20. GLEAN DOSE RESPONSE

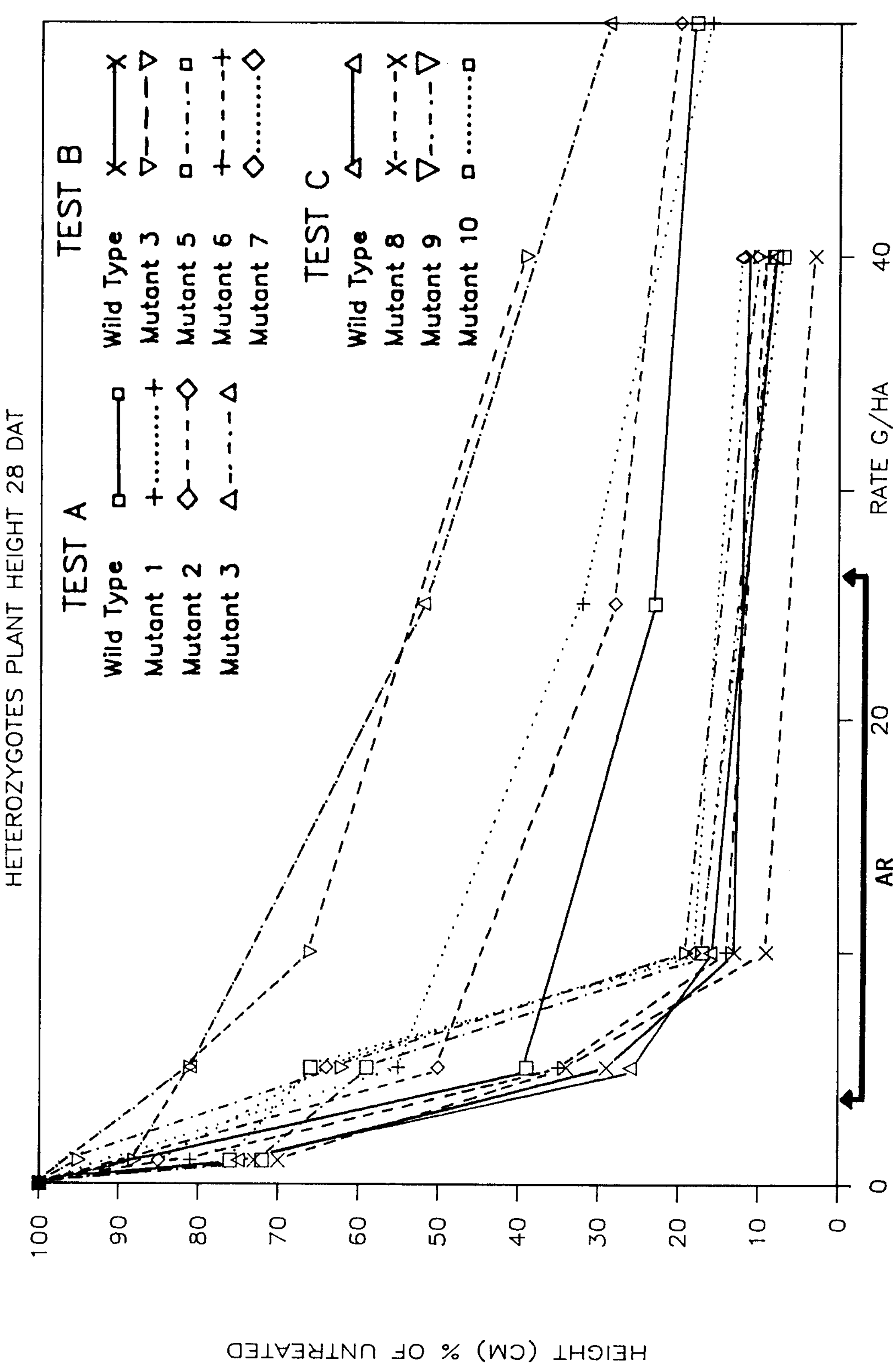

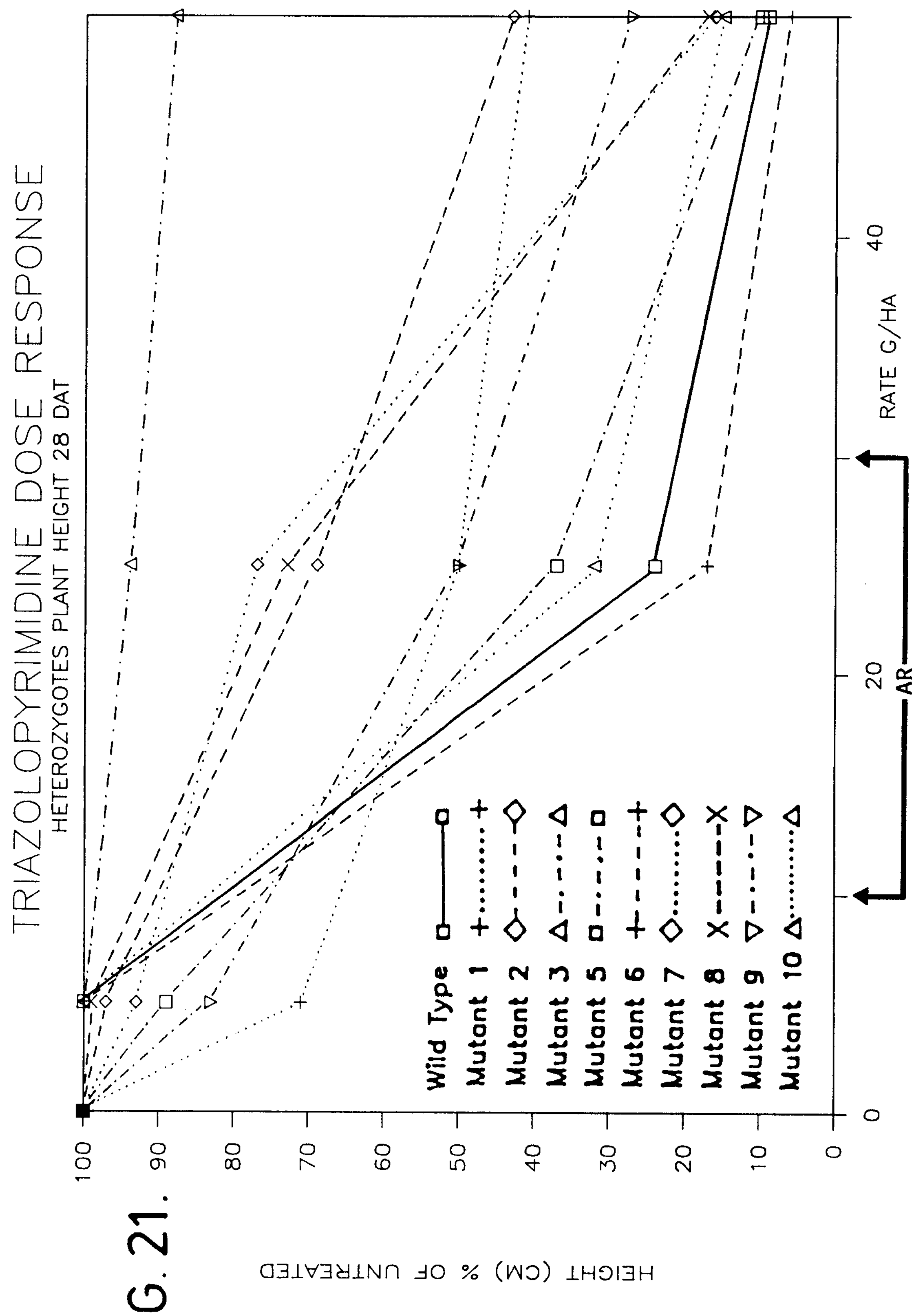
TRIAZOLOPYRIMIDINE DOSE RESPONSE
HETEROZYGOTES PLANT HEIGHT 28 DAT
HEIGHT (CM) % OF UNTREATED
100
90
80
70
60
50
40
30
20
10
0
0
20
40
RATE G/HA
AR
Wild Type
Mutant 1
Mutant 2
Mutant 3
Mutant 5
Mutant 6
Mutant 7
Mutant 8
Mutant 9
Mutant 10

FIG. 21.

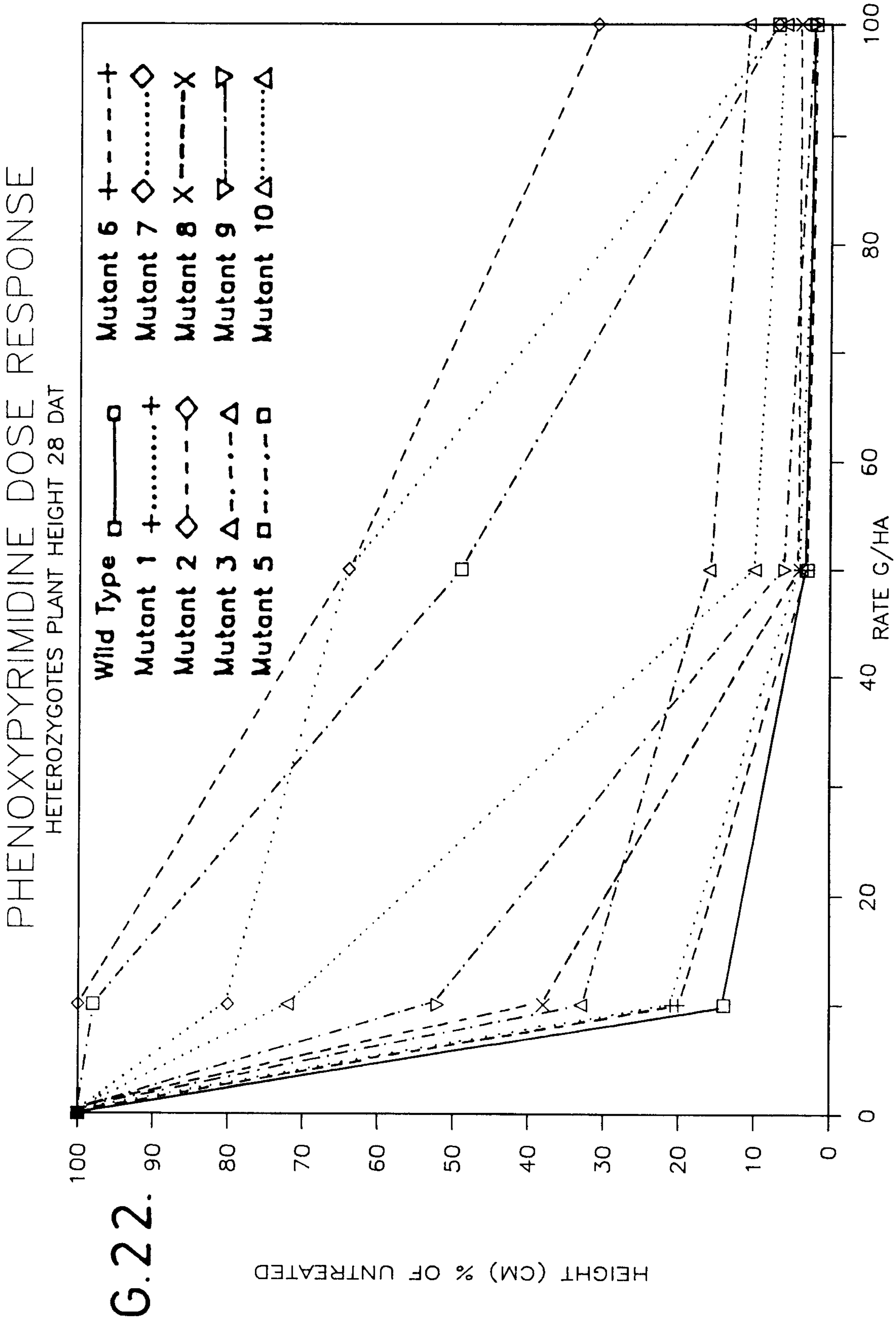
PHENOXYPYRIMIDINE DOSE RESPONSE
HETEROZYGOTES PLANT HEIGHT 28 DAT
Wild Type
Mutant 1
Mutant 2
Mutant 3
Mutant 5
Mutant 6
Mutant 7
Mutant 8
Mutant 9
Mutant 10
HEIGHT (CM) % OF UNTREATED
RATE G/HA
0
10
20
30
40
50
60
70
80
90
100
0
20
40
60
80
100

FIG.22.

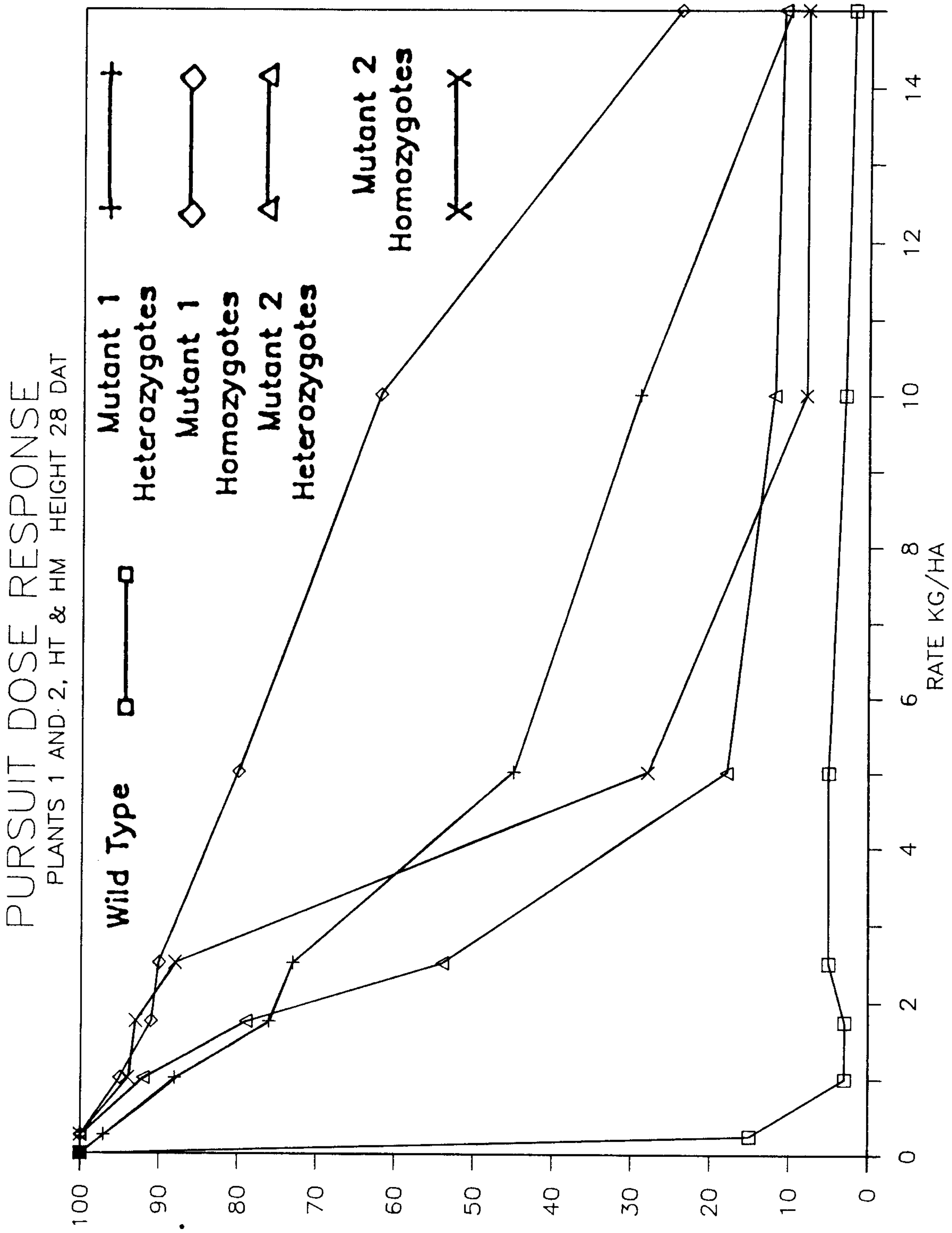
PURSUIT DOSE RESPONSE
PLANTS 1 AND 2, HT & HM HEIGHT 28 DAT
Wild Type
Mutant 1 Heterozygotes
Mutant 1 Homozygotes
Mutant 2 Heterozygotes
Mutant 2 Homozygotes
RATE KG/HA
0
2
4
6
8
10
12
14
100
90
80
70
60
50
40
30
20
10
0
HEIGHT % OF UNTREATED

FIG. 23.